# EUROPEAN PATENT APPLICATION

(11) **EP 4 741 391 A1**
(43) Date of publication of application: **13.05.2026**
(21) Application number: 24835281.7
(22) Date of filing: 27.06.2024
(51) Int. Cl.: C07D 471/04, C07D 403/12, C07D 409/12, C07D 409/14, C07D 413/12, C07D 401/14, C07D 487/02, A61K 31/437, A61P 3/10, A61P 25/00, A61P 31/12, A61P 33/00, A61P 35/00

(54) **NMT INHIBITOR, AND PREPARATION METHOD THEREFOR AND USE THEREOF**

(30) Priority: 05.07.2023 CN 202310819130
(71) Applicant: Nanjing Synnocare Pharmaceutical Technology Co., Ltd., Nanjing, Jiangsu 211122 (CN); Tianjin Synnocare Biomedical Technology Co., Ltd., Binhai New Area, Tianjin 300457 (CN)
(72) Inventor: FAN, Houxing, Nanjing, Jiangsu 211122 (CN); JIANG, Qiangqiang, Nanjing, Jiangsu 211122 (CN); WANG, Tiancai, Nanjing, Jiangsu 211122 (CN)
(74) Representative: Lavoix
(86) International application number: PCT/CN2024/101995
(87) International publication number: WO 2025/007800

(57) **Abstract**

Provided in the present invention are an NMT inhibitor, and a preparation method therefor and the use thereof. Specifically, the present invention relates to a compound as represented by formula (1) and a preparation method therefor, and a pharmaceutical composition containing the compound as represented by formula (1) and/or a pharmaceutically acceptable salt thereof, and a preparation method therefor and the use thereof as an NMT inhibitor in the preparation of an anti-tumor drug.

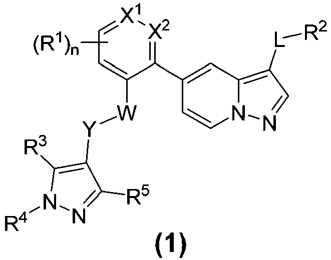

## Description

### FIELD OF THE INVENTION

The invention belongs to the field of medicinal chemistry, and more specifically, relates to a kind of NMT inhibitors as shown in Formula (1), and preparation methods therefor, as well as the use of these compounds for preparing pharmaceutial compositions for treating, regulating, and/or preventing diseases mediated by NMT.

### BACKGROUND

NMT (N-myristoyltransferase) is an enzyme that catalyzes the myristoylation of over 100 proteins in human cells and affects downstream related signaling pathways. In humans, protein myristoylation is mediated by two ubiquitously expressed N-myristoyltransferases, NMT1 and NMT2. Myristoylation is a co- and post-translational modification reaction in eukaryotes, where, through the action of NMT1 and NMT2, a myristoyl group is transferred to the N-terminal glycine of substrate proteins [Cell Death and Disease, 2018, 9 (12): 1143]. It acts like a "switch", triggering various reversible protein-membrane and protein-protein interactions.

NMT1 is highly expressed in various tumor tissues. In liver cancer, NMT1 is associated with poor prognosis; patients with high NMT1 expression have shorter survival times compared to those with low expression. The expression of NMT1 holds significant clinical importance. Detection in primary breast cancer tissues and adjacent tissues from 20 breast cancer patients showed that NMT1 expression was significantly higher in breast cancer tissues compared to adjacent tissues, particularly markedly elevated in triple-negative breast cancer tissues [Cell Death and Disease, 2018, 9 (12): 1143]. Elevated NMT1 is associated with tumor development and shorter patient survival. In colorectal cancer patients, NMT1 expression increased in peripheral blood and bone marrow and can serve as a diagnostic marker for colorectal cancer [J Transl Med. 2007, 5: 58]. High NMT1 expression is associated with poor prognosis in ovarian cancer and with the progression of liver cancer, brain cancer, etc. [Annals of medicine, 2023, 55 (1): 1422-1430].

NMT1 catalyzes the formation of myristoylated proteins from its substrate proteins, activating downstream signaling pathways such as NF-κB, C-Myc, and ERK, thereby promoting tumor cell proliferation, migration, and metastasis. Inhibiting NMT1 can induce cell cycle arrest and suppress cell proliferation and malignant growth [Annals of medicine, 2023, 55 (1): 1422-1430]. NMT1 is highly expressed in various tumors and is associated with tumor stage, prognosis, and survival, making it a potential target for precision cancer therapy. NMT inhibitors can significantly inhibit hematological tumor cells, including B lymphocyte activity, inhibit myristoylation in lymphoma cells, and suppress B-cell receptor signaling, leading to cell death and inhibiting tumor growth in mouse tumor models [Nature communications, 2020, 11 (1): 5348].

Although some small molecule NMT inhibitors have been disclosed, there remains a need to develop novel compounds with better activity and pharmacokinetic properties. The NMT inhibitor compounds of the present invention, having the structure represented by general formula (1), demonstrate excellent activity and profiles, potentially providing new options for precision cancer therapy.

### SUMMARY OF THE INVENTION

The objective of the present invention is to provide a novel NMT inhibitor with potent activity, as well as preparation therefor and uses thereof.

In the first aspect of the present invention, it provides a compound as shown in Formula (1), or an isomer, crystal form, pharmaceutically acceptable salt, hydrate, or solvate thereof: 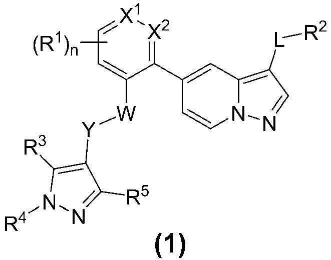 wherein:
n is 0, 1, 2 or 3;
X¹ and X² are each independently selected from the group consisting of: CH, CR¹ or N;
W is selected from the group consisting of: a bond, O, S or NH;
Y is selected from the group consisting of: a bond, -(CH₂)ₘ-, -(CH₂)ₘO- or -(CH₂)ₘO-(CH₂)ₘ, wherein m is 1, 2 or 3;
L is -(CH₂)ₘ-;
each R¹ is independently selected from the group consisting of: D, hydroxyl, halogen, CN, NO₂, NH₂, C₁₋₄ alkyl, C₂₋₄ alkenyl, C₂₋₄ alkynyl, C₁₋₄ haloalkyl, C₁₋₄ alkoxy, C₁₋₄ haloalkoxy, C₃₋₆ saturated or partially unsaturated carbocyclic group, 3-8-membered saturated or partially unsaturated heterocyclyl, C₆₋₁₀ aryl or 5-12 membered heteroaryl;
R² is -NR⁶R⁷, wherein R⁶ and R⁷ are each independently selected from the group consisting of: H, D, C₁₋₆ alkyl or C₁₋₆ haloalkyl;
or, R⁶ and R⁷ are taken together with the attached nitrogen atom to form a 3-8-membered saturated or partially unsaturated heterocyclyl, or a 5-12 membered heteroaryl;
R³ is selected from the group consisting of: H, D, C₁₋₄ alkyl, C₁₋₄ haloalkyl, C₁₋₄ alkoxy, C₁₋₄ haloalkoxy, C₃₋₆ saturated or partially unsaturated carbocyclic group, 3-8-membered saturated or partially unsaturated heterocyclyl, C₆₋₁₀ aryl or 5-12 membered heteroaryl;
R⁴ is selected from the group consisting of: C₁₋₄ alkyl, C₁₋₄ haloalkyl, C₃₋₆ saturated or partially unsaturated carbocyclic group, 3-8-membered saturated or partially unsaturated heterocyclyl, C₆₋₁₀ aryl or 5-12 membered heteroaryl;
R⁵ is selected from the group consisting of: hydroxyl, CN, C₁₋₆ alkyl, C₁₋₆ haloalkyl, hydroxyl substituted C₁₋₆ alkyl, cyano substituted C₁₋₆ alkyl or -C(O)NR⁸R⁹; wherein R⁸ and R⁹ are each independently selected from the group consisting of: H, D, C₁₋₆ alkyl or C₁₋₆ haloalkyl;
or, R⁸ and R⁹ are taken together with the attached nitrogen atom to form a 3-8-membered saturated or partially unsaturated heterocyclyl, or a 5-12 membered heteroaryl;
wherein, the alkyl, alkenyl, alkynyl, haloalkyl, alkoxy, haloalkoxy, carbocyclic group, heterocyclyl, aryl and heteroaryl are each optionally substituted by one or more substituents selected from the group consisting of: D, hydroxyl, halogen, CN, NO₂, NH₂, C₁₋₄ alkyl, C₂₋₄ alkenyl, C₂₋₄ alkynyl, C₁₋₄ haloalkyl, C₁₋₄ alkoxy or C₁₋₄ haloalkoxy.

In another preferred embodiment, wherein each R¹ is independently selected from the group consisting of: F, Cl, Br, CN, NO₂, C₁₋₃ alkyl, C₁₋₃ haloalkyl, C₁₋₃ alkoxy or haloalkoxy.

In another preferred embodiment, wherein each R² is -NR⁶R⁷, wherein R⁶ and R⁷ are each independently selected from the group consisting of: H, D, C₁₋₃ alkyl or C₁₋₃ haloalkyl.

In another preferred embodiment, wherein R³ is selected from the group consisting of: H, D, C₁₋₃ alkyl or C₁₋₃ haloalkyl.

In another preferred embodiment, wherein R⁴ is selected from the group consisting of: C₁₋₃ alkyl or C₁₋₃ haloalkyl.

In another preferred embodiment, wherein R⁵ is selected from the group consisting of: CN, C₁₋₆ alkyl, C₁₋₆ haloalkyl, hydroxyl substituted C₁₋₆ alkyl, cyano substituted C₁₋₆ alkyl or -C(O)NR⁸R⁹;
wherein R⁸ and R⁹ are each independently selected from the group consisting of: H, D, C₁₋₃ alkyl or C₁₋₃ haloalkyl.

In another preferred embodiment, wherein the compound has a structure selected from the group consisting of:

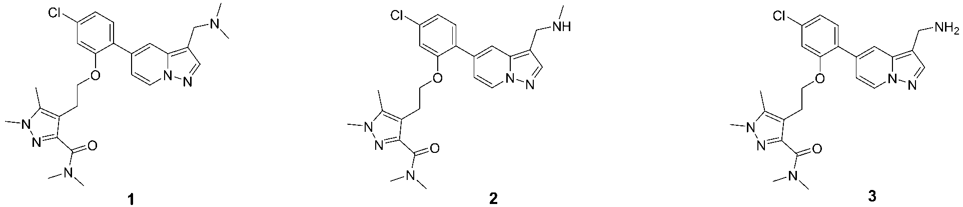

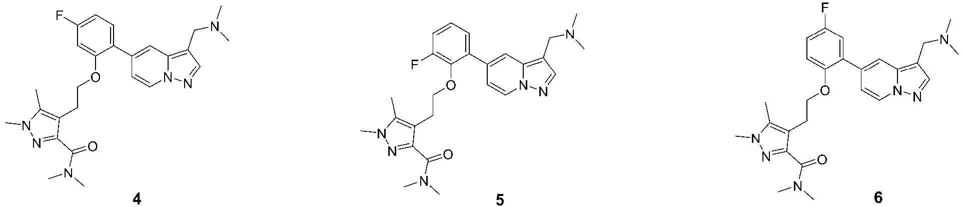

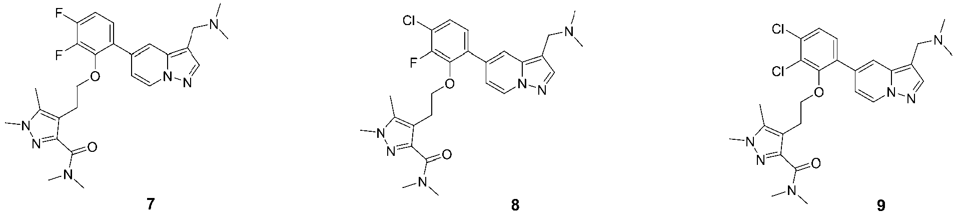

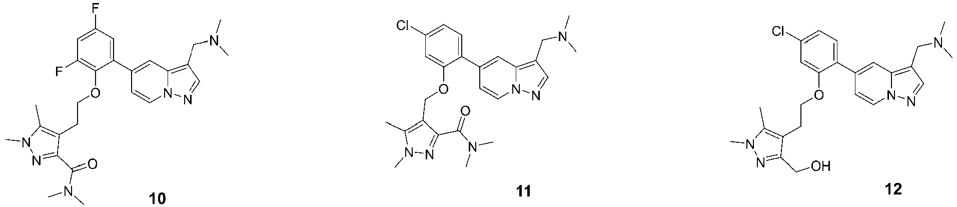

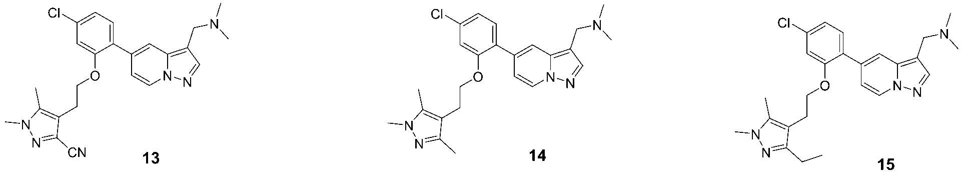

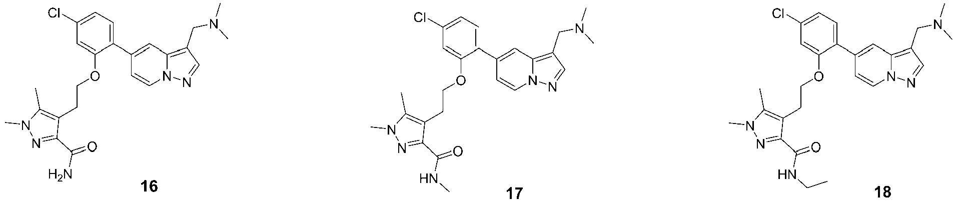

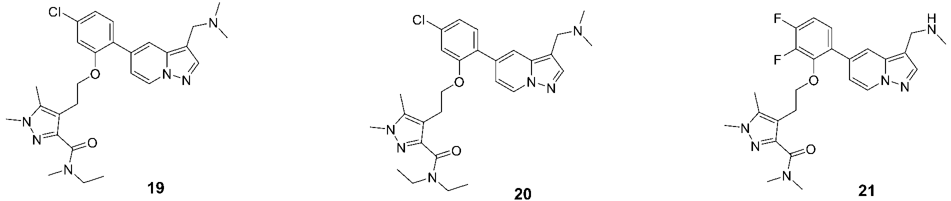

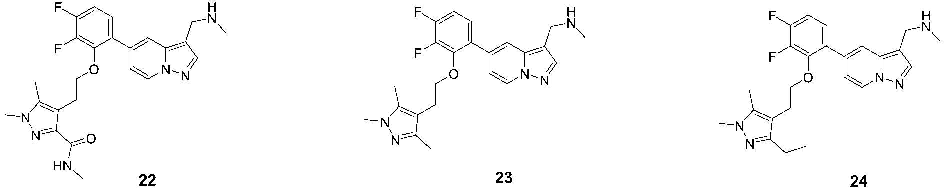

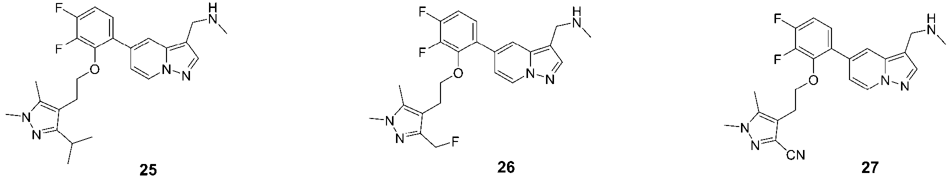

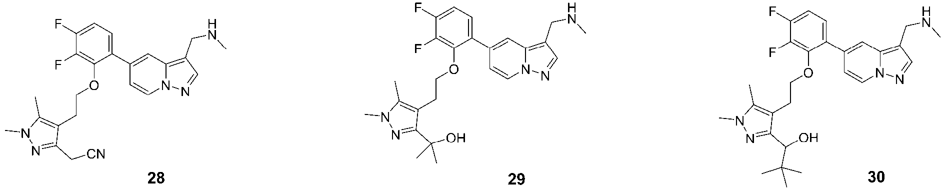

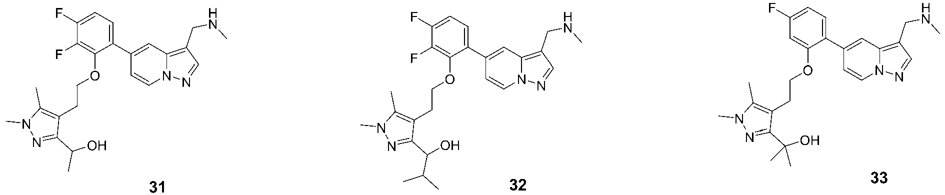

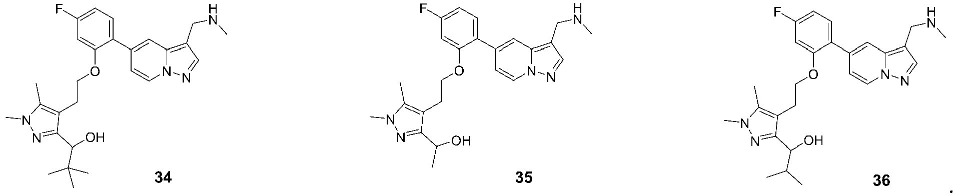

In the second aspect of the present invention, it provides a pharmaceutical composition for treating, regulating, and/or preventing a disease associated with NMT, wherein the pharmaceutical composition comprises:
(1) the compound described in the first aspect of the present invention as an active ingredient, or an isomer, crystal form, pharmaceutically acceptable salt, hydrate, or solvate thereof; and
optionally (2) a pharmaceutically acceptable excipient, or carrier.

In the third aspect of the present invention, it provides use of the compound as described in the first aspect of the present invention, or an isomer, crystal form, pharmaceutically acceptable salt, hydrate, or solvate thereof, or the pharmaceutical composition as described in the second aspect of the present invention for preparing a pharmaceutical composition for treating, regulating, and/or preventing a disease mediated by NMT.

In another preferred embodiment, wherein the related disease mediated by NMT include infectious disease or hyperproliferative disease.

In another preferred embodiment, wherein the infectious disease includes protozoan infection, such as malaria and leishmaniasis.

In another preferred embodiment, wherein the infectious disease includes viral infection, such as human rhinovirus infection and HIV infection.

In another preferred embodiment, wherein the hyperproliferative disease is selected from the following groups: lymphoma, leukemia, brain tumor, gastric tumor, liver cancer, lung cancer, intestinal cancer, pancreatic cancer, breast cancer, cervical cancer, ovarian cancer, endometrial cancer and prostate cancer.

It should be understood that within the scope of the present invention, all the technical features of the present invention and those specifically described in the following text (e.g., examples) can be combined with each other to form new or preferred technical solutions. Due to the limited space, we will not enumerate them one by one here.

### DETAILED DESCRIPTION OF THE ILLUSTRATED EMBODIMENTS

The inventor, after extensive and intensive research, and through a large number of screenings and tests, first discovered a kind of compounds of Formula (1), which have a significant therapeutic effect on diseases mediated by NMT. The compounds of the present invention have a potent inhibitory activity against NMT1 enzyme and good cell proliferation inhibition activity against MV-4-11 cell line. On this basis, the present invention was completed.

### THE COMPOUND OF THE PRESENT INVENTION AND ITS SYNTHESIS

The present invention provides an inhibitor of NMT, i.e., a compound of Formula (1), or an isomer, crystal form, pharmaceutically acceptable salt (inorganic salt or organic salt), hydrate, or solvate thereof. Preferably, the compound of the present invention is as described in the first aspect of the present invention.

The present invention also provides a method for preparing the compound of Formula (1) of the present invention. The following specifically describes the method for preparing the compound of Formula (1) of the present invention, but these specific methods do not constitute any limitation on the present invention.

In one aspect, the compounds described herein are prepared according to the well-known methods. However, the conditions of the process, such as reactants, solvents, bases, amounts of compounds used, reaction temperature, time required for the reactions, etc., are not limited to the following explanations. The compounds of the present invention may also be conveniently prepared, optionally in combination with various synthesis methods described in this specification or well-known in the art, and such combinations are readily carried out by those skilled technicians in the art. On the other aspect, the present invention also provides the preparation methods of the compounds represented by the general formula (1), which is prepared by the following general scheme 1:

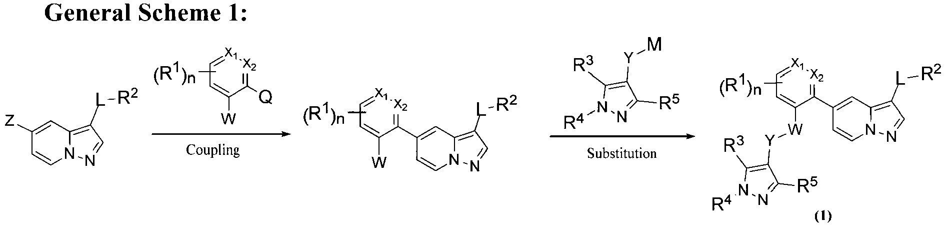

The embodiments of the compound of Formula (1) can be prepared according to the general scheme 1, wherein Z is a halogen, Q is a boronic acid group or boronic ester group, M is a halogen, Oms or OTos, and R¹, R², R³, R⁴, R⁵, X¹, X², L, W, Y and n are as defined above.

### Related definitions

Unless otherwise specified, the following terms and phrases used herein are intended to have the following meanings. A specific term or phrase should not be considered indefinite in the absence of a particular definition, but should be understood according to its ordinary meaning. When a trade name appears herein, it is intended to refer to its corresponding commercial product or active ingredient thereof.

The term "pharmaceutically acceptable" used herein in terms of those compounds, materials, compositions, and/or dosage forms, which are suitable for use in contact with human and animal tissues within the scope of reliable medical judgment, without excessive toxicity, irritation, allergic reactions or other problems or complications, commensurate with a reasonable benefit/risk ratio.

The term "pharmaceutically acceptable salt" refers to a salt of the compound of the present disclosure that is prepared by reacting the compound having a specific substituent of the present invention with a relatively non-toxic acid or base. When the compound of the present disclosure contains a relatively acidic functional group, a base addition salt can be obtained by contacting the compound with a sufficient amount of a base in a pure solution or a suitable inert solvent. The pharmaceutically acceptable base addition salts include a salt of sodium, potassium, calcium, ammonium, organic amine or magnesium, or similar salts. When the compound of the present disclosure contains a relatively basic functional group, an acid addition salt can be obtained by contacting the compound with a sufficient amount of acid in a pure solution or a suitable inert solvent. Examples of pharmaceutically acceptable acid addition salts include an inorganic acid salt, wherein the inorganic acid include, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphinic acid and the like; and an organic acid salt, wherein the organic acid includes, for example, acetic acid, propionic acid, isobutyric acid, maleic acid, malonic acid, benzoic acid, succinic acid, octanedioic acid, trans-butenedioic acid, lactic acid, mandelic acid, phthalic acid, benzenesulfonic acid, p-toluenesulfonic acid, citric acid, tartaric acid, and methanesulfonic acid, and the like; and salts of amino acids (such as arginine and the like), and a salt of organic acids such as glucuronic acid and the like. Certain specific compounds of the present invention contain both basic and acidic functional groups, thus can be converted to any base or acid addition salt.

The pharmaceutically acceptable salt of the present disclosure can be prepared from the parent compound that contains an acid or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereoisomers, (D)-isomers, (L)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term "cis-trans isomer" or "geometric isomer" is caused by the inability to rotate freely double bonds or single bonds of ring -forming carbon atoms.

Unless otherwise specified, the term "diastereomer" refers to a stereoisomer in which a molecule has two or more chiral centers and the relationship between the molecules is not mirror images.

Unless otherwise specified, *"(D)"* or " (+)" refers to dextrorotation, *"(L)"* or " (-)" refers to levorotation, and *"(DL)"* or " (±)" refers to racemic.

Unless otherwise specified, the absolute configuration of a stereogenic center is represented by a wedged solid bond ( 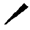 ) and a wedged dashed bond ( 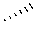 ), and the relative configuration of a stereogenic center is represented by a straight solid bond ( 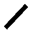 ) and a straight dashed bond ( 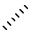 ), a wave line ( 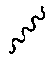 ) is used to represent a wedged solid bond ( 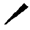 ) or a wedged dashed bond ( 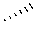 ), or the wave line ( 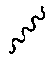 ) is used to represent a straight solid bond ( 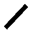 ) or a straight dashed bond ( 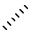 ).

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, then the isomer or enantiomer excess (ee value) is 80%.

Optically active (R)- and (S)-isomers, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as an amino) or an acidic functional group (such as a carboxyl), the compound reacts with an appropriate optically active acid or base to form a salt of the diastereomeric isomer which is then subjected to diastereomeric separation through the conventional method in the art to give the pure enantiomer. In additional, the enantiomers and diastereoisomer are generally isolated through chromatography which uses a chiral stationary phase and optionally combines with a chemical derivative method (such as carbamate generated from amine).

Compounds disclosed herein may contain unnatural proportions of atomic isotopes at one or more of the atoms that make up the compounds. For example, a compound may be labeled with a radioisotope such as tritium (³H), iodine-125 (¹²⁵I) or C-14 (¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs usually have the advantages of reduced toxic side effects, increased drug stability, enhanced efficacy, and prolonged biological half-life of the drug. All changes in the isotopic compositions of compounds disclosed herein, regardless of radioactivity are included within the scope of the present disclosure.

The term "optional" or "optionally" means that the subsequent event or condition may occur but not requisite, that the term includes the instance in which the event or condition occurs and the instance in which the event or condition does not occur.

The term "substituted" refers to that one or more than one hydrogen atoms on a specific atom are substituted by a substituent, including deuterium and hydrogen variants, as long as the valence of the specific atom is normal and the substituted compound is stable. When the substituent is oxo (i.e., =O), it means that two hydrogen atoms are substituted. Positions on an aromatic ring cannot be substituted by oxo. The term "optionally substituted" means an atom can be substituted with a substituent or not, unless otherwise specified, the type and the number of the substituent may be arbitrary so long as being chemically achievable.

When any variable (e.g., R) occurs in the constitution or structure of the compound more than once, the definition of the variable at each occurrence is independent. Thus, for example, if a group is substituted by 0-2 R, the group can be optionally substituted by up to two R, wherein the option of R at each occurrence is independent. Moreover, the combination of the substituent and/or the variant thereof is allowed only when the combination results in a stable compound.

When the number of a linking group is 0, such as -(CH₂)₀-, it means that the linking group is a single bond.

When one of the variables is a single bond, it means that the two groups linked by the single bond are connected directly. For example, when L in X-L-Y represents a single bond, the structure of X-L-Y is actually X-Y.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁ and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.; Similarly, n membered to n+m membered indicates the number of atoms on a ring is n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, etc.

Unless otherwise specified, "C₁₋₆ alkyl" refers to a linear or branched saturated hydrocarbon group composed of 1 to 6 carbon atoms. The C₁₋₆ alkyl includes C₁₋₂, C₁₋₃, C₁₋₄, C₁₋₅, C₂₋₄, C₂₋₆, C₃₋₅, C₅ and C₆ alkyl and the like; it can be monovalent (such as methyl), divalent (such as methylene), or multivalent (such as methine). Examples of C₁₋₆ alkyl include but not limited to methyl, ethyl, propyl, n-propyl, isopropyl, n-butyl, isobutyl, tert butyl, sec butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2,3-dimethylbutyl, 2-ethylbutyl, 2-methylpentyl, 3-methylpentyl, 4-methylpentyl, and their various branched isomers and the like.

Unless otherwise specified, "C₁₋₃ alkyl" refers to a linear or branched saturated hydrocarbon group composed of 1 to 3 carbon atoms. The C₁₋₃ alkyl includes C₁₋₂ and C₂₋₃ alkyl and the like, it can be monovalent (such as methyl), divalent (such as methylene), or multivalent (such as methine). Examples of C₁₋₃ alkyl include but not limited to methyl, ethyl, propyl, n-propyl, isopropyl and the like.

Unless otherwise specified, "C₁₋₃ alkoxy" refers to an alkyl containing 1 to 3 carbon atoms that are connected to the rest of the molecule through an oxygen atom. The C₁₋₃ alkoxy includes C₁₋₂, C₂, and C₃ alkoxy and the like. Examples of C₁₋₃ alkoxy include but are not limited to methoxy, ethoxy, propoxy, n-propyloxy, isopropyl and the like.

The term "halo" or "halogen" refers to fluorine, chlorine, bromine and iodine atom.

The term "hydroxyl" refers to -OH.

The term "cyano" refers to -CN.

### SPECIFIC PHARMACEUTICAL AND MEDICAL TERMS

The term "acceptable," as used herein, means that a prescription component or active ingredient does not have excessively harmful effects on the health of subject.

As used herein, the term "treatment," "course of treatment" or "therapy" includes alleviating, inhibiting or improving symptoms or conditions of diseases; inhibiting the occurrence of complications; improving or preventing potential metabolic syndrome; suppressing the occurrence of diseases or symptoms, such as controlling the development of diseases or conditions; alleviating diseases or symptoms; reducing disease or symptoms; alleviating complications caused by diseases or symptoms, or preventing or treating symptoms caused by diseases or symptoms. As used herein, a certain compound or pharmaceutical composition, which can improve a certain disease, symptom or condition after administration, especially reduce its severity, delay the onset, slow down the progress of the disease, or shorten the duration of the disease. Whether fixed administration or temporary administration, continuous administration or intermittent administration, which can be attributed to related administration.

The term "active ingredient" refers to the compounds represented by the general formula (1) and the pharmaceutically acceptable inorganic or organic salts of the compounds of the general formula (1). Compounds of the present invention may contain one or more asymmetric centers, and thus appear in the form of racemates, racemic mixtures, single enantiomers, diastereomeric compounds or single diastereomers. The asymmetric centers that can exist depend on the properties of various substituents on the molecule. Each such asymmetric center will independently produce two optical isomers, and all possible optical isomers, diastereomer mixtures and pure or partially pure compounds are included in the scope of the present disclosure. The present disclosure is meant to include all such isomeric forms of these compounds.

The terms such as "compound," "composition," "agent" or "medicine or medicament" can be used interchangeably here, and all refer to a compound or composition which can induce the desired pharmaceutical and/or physiological response through local and/or systemic action when administered to a person (human or animal).

The term "administered, administering or administration" here refers to the direct administration of the compounds or compositions, or the administration of prodrugs, derivatives or analogs of the active compounds.

Although the numerical ranges and parameters used to define the wide range of the present invention are approximate values, the relevant values in the specific embodiments have been presented here as accurately as possible. However, any numerical value inevitably contains standard deviation caused by individual test methods. Here, "about" usually means that the actual value is within plus or minus 10%, 5%, 1% or 0.5% of a specific value or range. Alternatively, the word "about" means that the actual value falls within the acceptable standard error of the average value, depending on the consideration of those skilled in the art. Except for experimental examples, or unless otherwise specified, it is understood that all ranges, quantities, values and percentages used herein (for example, to describe the amount of materials, the length of time, the temperatures, the operating conditions, the proportions of quantities and other similar ones) are modified by "about". Therefore, unless otherwise stated, the numerical parameters disclosed in this specification and the appended claims are approximate values, and can be changed as required. At least these numerical parameters should be understood as the indicated effective digits and the numerical values obtained by applying the general carry method.

Unless otherwise defined in this specification, the meanings of scientific and technical terms used herein are the same as those understood and used by those skilled in the art. In addition, the singular noun used in this specification covers the plural form of the noun without conflict with the context; the plural nouns used also cover the singular form of the noun.

### ROUTE OF ADMINISTRATION

The compound of the present invention and its pharmaceutically acceptable salts can be made into various preparations, which contain the compound of the present invention or its pharmaceutically acceptable salts and pharmaceutically acceptable excipients or carriers in a safe and effective amount range. Among them, "safe and effective amount" means that the amount of the compound is capable of obviously improving the condition without causing serious side effects. The safe and effective dose of the compound is determined according to the age, illness, course of treatment and other specific conditions of the subject.

"Pharmaceutically acceptable excipient or carrier" refers to one or more compatible solid or liquid fillers or gel substances, which are suitable for human use and must have sufficient purity and low toxicity. "Compatibility" here means that each component in the composition can be mixed with the compounds of the present invention and between them, without significantly reducing the efficacy of the compound. Examples of pharmaceutically acceptable excipients or carriers include cellulose and its derivatives (such as sodium carboxymethyl cellulose, sodium ethyl cellulose, cellulose acetate, etc.), gelatin, talc, solid lubricants (such as stearic acid and magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agent, flavoring agent, stabilizer, antioxidant, preservative, pyrogen-free water, etc.

The compounds of the present invention can be administered orally, rectally, parenterally (intravenously, intramuscularly or subcutaneously) or topically.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with: (a) a filler or compatibilizer, such as starch, lactose, sucrose, glucose, mannitol, and silicic acid; (b) binders such as hydroxymethyl cellulose, alginates, gelatin, polyvinyl pyrrolidone, sucrose and acacia; (c) humectant, such as glycerol; (d) disintegrant such as agar, calcium carbonate, potato or tapioca starch, alginic acid, certain complex silicates, and sodium carbonate; (e) slow solvents, such as paraffin; (f) an absorption accelerator, such as a quaternary amine compound; (g) wetting agents, such as cetyl alcohol and glyceryl monostearate; (h) adsorbent, such as kaolin; and (i) a lubricant such as talc, calcium stearate, magnesium stearate, solid polyethylene glycol, sodium lauryl sulfate, or mixtures thereof. In capsule, tablets and pill, the dosage form may also contain a buffer.

Solid dosage forms such as tablets, sugar pills, capsules, pills and granules may be prepared using coatings and shell materials such as casings and other materials well-known in the art. They may comprise an opacifying agent and the release of the active compound or compound in such a composition may be released in a delayed manner in a portion of the digestive tract. Examples of embedding components that may be used are polymeric substances and waxes. If desired, the active compound may also form microcapsules with one or more of the above excipients.

Liquid dosage forms for oral administration include pharmaceutically acceptable emulsions, solutions, suspensions, syrups or tinctures. In addition to the active compound, the liquid dosage form may comprise inert diluents conventionally used in the art, such as water or other solvents, solubilizers and emulsifiers, for example ethanol, isopropanol, ethyl carbonate, ethyl acetate, propylene glycol, 1,3-butanediol, dimethylformamide and oils, particularly cottonseed oil, peanut oil, corn germ oil, olive oil, castor oil, sesame oil or mixtures of these and the like.

In addition to these inert diluents, the composition may also contain adjuvants such as wetting agents, emulsifiers and suspending agents, sweeteners, flavoring agents and flavorants.

In addition to the active compounds, the suspension may comprise suspending agents such as ethoxylated isostearyl alcohol, polyoxyethylene sorbitol and sorbitan esters, microcrystalline cellulose, aluminum methoxide, agar or mixtures of these and the like.

The composition for parenteral injection may comprise a physiologically acceptable sterile aqueous or non-aqueous solution, dispersion, suspension or emulsion, and a sterile powder for reconstitution into a sterile injectable solution or dispersion. Suitable aqueous and non-aqueous carriers, diluents, solvents or excipients include water, ethanol, polyols and suitable mixtures thereof.

Dosage forms of the compounds of the present invention for topical administration include ointments, powders, patches, sprays and inhalants. The active ingredient is mixed under sterile conditions with a physiologically acceptable carrier and any preservatives, buffers, or propellants as may be required.

The compound of the present invention may be administered alone or in combination with other pharmaceutically acceptable compounds.

When a pharmaceutical composition is used, a safe and effective amount of a compound of the present invention is applied to a mammal (e.g., a human) in need of treatment, wherein the dose at the time of administration is a pharmaceutically acceptable effective dose, and for a human of 60 kg body weight, the daily dose is generally 1 to 2000 mg, preferably 50 to 1000 mg. Of course, specific dose should also take into account factors such as route of administration, patient health, etc., which are within the skill scope of a skilled physician.

The main advantages of the present disclosure include:
(a) Unexpectedly, the compound of Formula (1) of the present disclosure showed excellent *in vitro* activity, including potent inhibitory activity against NMT1 enzyme and good cell proliferation inhibition activity against MV-4-11 cell line, making it more suitable for development as a drug.
(b) The compound of the present disclosure showed good druggablility.

The above features mentioned in the present disclosure, or the features mentioned in the examples, may be combined at random. All of the features disclosed in this specification may be used in any composition form and the various features disclosed in the specification may be replaced with any alternative feature that provides the same, equivalent, or similar purpose. Thus, unless otherwise specified, the disclosed features are merely generic examples of equivalent or similar features.

Various specific aspects, features and advantages of the above compounds, methods, and pharmaceutical compositions will be set forth in detail as following. It is to be understood that the following detailed description and examples describe specific embodiments for reference only. Various changes or modifications may occur to those skilled in the art after reading the description of the invention, and such equivalents fall within the scope of the application.

The structures of compounds disclosed herein can be confirmed by conventional methods well known to those skilled in the art. If the present disclosure relates to an absolute configuration of a compound, the absolute configuration can be confirmed by conventional techniques in the field. For example, single crystal X-ray diffraction (SXRD), the diffraction intensity data of the cultivated single crystal is collected using a Bruker D8 venture diffractometer with a light source of CuKα radiation in a scanning mode of φ/ω scan. After collecting the relevant data, the crystal structure is further analyzed by the direct method (Shelxs97) to conform the absolute configuration.

The solvent used in the present invention can be obtained commercially. The compounds of the present disclosure are named according to the conventional naming principles in the art or by ChemDraw^{®} software, and the commercially available compounds use the supplier catalog names.

In all examples, ¹H-NMR was recorded with a Varian Mercury 400 NMR spectrometer and that chemical shift was expressed as δ (ppm); Silica gel for separation is 200-300 mesh without specific statement, and the ratio of eluents is volume ratio.
Abbreviations: (Boc)₂O represents di-tert-butyl dicarbonate; ACN (CH₃CN) represents acetonitrile; °C represents degree celsius; Cs₂CO₃ represents caesium carbonate; CD₃OD represents Methanol-*d*₄; EA (EtOAc) represents ethyl acetate; DCM represents Dichloromethane; DIEA (DIPEA) represents N,N-diisopropylethylamine; Dioxane represents 1,4-dioxane; DMF represents N,N-dimethylformamide; h represents hour; HATU represents *O*-(7-Azabenzotriazol-1-yl)-*N*,*N*,*N*',*N*'-tetramethyl uronium hexa fluorophosphate; H₂O₂ represents hydrogen peroxide; K₂CO₃ represents potassium carbonate; K₃PO₄ represents potassium phosphate tribasic; LC-MS represents liquid chromatography mass spectrometry; LiAlH₄ represents lithium aluminum hydride; MS represents mass spectrometry; MsCl represents methanesulfonyl chloride; NaBH₃CN represents sodium cyanoborohydride; NaHCO₃ represents sodium bicarbonate; NaOH represents sodium hydroxide; NIS represents N-iodosuccinimide; NMR represents nuclear magnetic resonance; Pd₂(dba)₃ represents tris(dibenzylideneacetone)dipalladium; Pd(dppf)₂Cl₂ represents [1,1'-Bis-(diphenylphosphino)ferrocene]dichloropalladium(II); PE represents petroleum ether; POCl₃ represents phosphorus oxychloride; TEA represents triethylamine_{∘}

The present invention is further described through the following examples. It should be understood that these examples are only used to illustrate the invention, but not intended to impose any limitations on the present invention. The experimental methods not specified in the following examples are usually carried out under conventional conditions, or according to the conditions recommended by the manufacturer. Unless otherwise stated, percentages and parts are weight percentages and weight parts.

### Preparation Example 1: Synthesis of 2-[3-(ethoxycarbonyl)-1,5-dimethylpyrazol-4-yl] ethyl methanesulfonate (Intermediate 1)

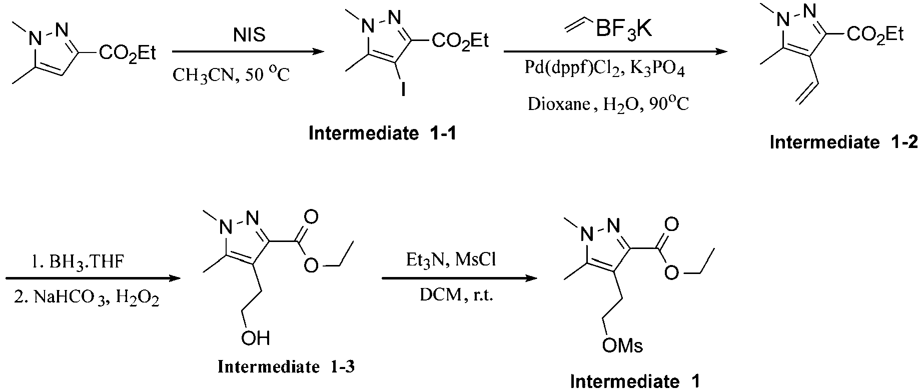

### Step 1: Synthesis of Intermediate 1-1

To a solution of ethyl 1,5-dimethyl-1H-pyrazole-3-carboxylate (5 g, 29.73 mmol) in ACN (100 mL) was added NIS (7.36 g, 32.70 mmol). The resulting mixture was stirred at 50 °C until the starting material is consumed. Acetonitrile was removed by direct concentration. The residue was poured into water (100 mL) and extracted with EA (50 mL *3). The organic phases were combined, dried, concentrated, and purified by column chromatography (PE/EA = 20/1 to 5/1) to afford **intermediate 1-1** as a yellow solid (8.21 g, yield 94%). ESI-MS m/z: 294.9 [M+H]⁺.

### Step 2: Synthesis of Intermediate 1-2

**Intermediate 1-1** (8.21 g, 27.93 mmol) and potassium vinyltrifluoroborate (4.11 g, 30.72 mmol) were dissolved in dioxane/water (100/10 mL). Pd(dppf)Cl₂ (2.04 g, 2.79 mmol) and K₃PO₄ (11.86 g, 55.86 mmol) were added, and the reaction mixture was stirred at 90 °C. The reaction progress was monitored by LC-MS, and the starting material was completely consumed. Dioxane was removed by direct concentration, and the residue was poured into water (100 mL) and extracted with EA (50 mL*3). The combined organic phases were dried, concentrated, and purified by column chromatography (PE/EA = 10:1 to 5:1) to afford **intermediate 1-2** as a white solid (4.86 **g,** yield 90%). ESI-MS m/z: 195.0 [M+H]⁺.

### Step 3: Synthesis of Intermediate 1-3

**Intermediate 1-2** (4.86 g, 25.05 mmol) was dissolved in THF (50 mL). BH₃·THF (1 M, 25.05 mL) was slowly added at 0 °C, and the reaction mixture was stirred at 0 °C for 3 h. Subsequently, 30% aqueous sodium bicarbonate solution (20 mL) and 30% hydrogen peroxide (20 mL) were added and stirred at 0 °C. The reaction progress was monitored by LC-MS, and the starting material was completely consumed. The mixture was poured into water (100 mL) and extracted with EA (50 mL*3). The combined organic phases were dried, concentrated, and purified by column chromatography (PE/EA = 5/1 to 1/1) to afford **intermediate 1-3** as a white solid (1.26 g, yield 24%). ESI-MS m/z: 213.0 [M+H]⁺.

### Step 4: Synthesis of Intermediate 1

**Intermediate 1-3** (1.26 g, 5.94 mmol) and TEA (1.20 g, 11.89 mmol) were dissolved in DCM (30 mL). MsCl (748 mg, 6.53 mmol) was added under argon protection with stirring at 0 °C. After the addition was complete, the reaction mixture was stirred at room temperature. The reaction progress was monitored by LC-MS, and the starting material was completely consumed. The mixture was poured into water (50 mL) and extracted with DCM (30 mL*3). The combined organic phases were dried, concentrated, and purified by column chromatography (PE/EA = 10:1 to 2:1) to afford **intermediate 1** as a yellow solid (1.59 g, yield 92%). ESI-MS m/z: 291.0 [M+H]⁺.

**Intermediate 2-4** can be synthesized by a method similar to intermediate 1.

| Compound | Structure | Name | ESI-MS: [M+H]⁺ |
|---|---|---|---|
| **Intermediate 2** | 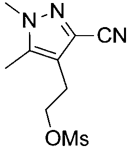 | 2-(3-cyano-1,5-dimethylpyrazol -4-yl)ethyl methanesulfonate | 244.0 |
| **Intermediate 3** | 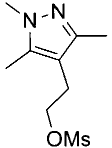 | 2-(1,3,5-trimethylpyrazol-4-yl)e thyl methanesulfonate | 233.1 |
| **Intermediate 4** | 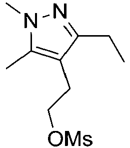 | 2-(3-ethyl-1,5-dimethylpyrazol-4-yl)ethyl methanesulfonate | 247.1 |

### Preparation Example 2: Synthesis of ethyl 4-(chloromethyl)-1,5-dimethylpyrazole-3-carboxylate (Intermediate 5)

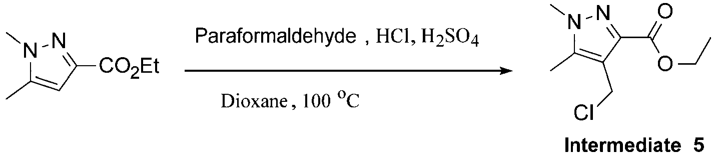

### Step 1: Synthesis of Intermediate 5

Ethyl 1,5-dimethyl-1H-pyrazole-3-carboxylate (2 g, 11.90 mmol) and paraformaldehyde (714 mg, 23.81 mmol) were dissolved in 1,4-dioxane (30 mL). Concentrated hydrochloric acid (12 M, 2 mL) and sulfuric acid (119 mg, 1.19 mmol, 98%) were added, and the reaction mixture was stirred at 100 °C. The reaction progress was monitored by LC-MS, and the starting material was completely consumed. 1,4-dioxane was removed by direct concentration, and the residue was poured into water (100 mL) and extracted with EA (50 mL*3). The combined organic phases were dried, concentrated, and afforded **intermediate** 5 as a yellow solid (8.21 g, yield 94%). ESI-MS m/z: 217.0 [M+H]⁺.

### Preparation Example 3: Synthesis of 2-(1,5-dimethyl-3-(prop-2-yl)pyrazol-4-yl]ethyl methanesulfonate (Intermediate 6)

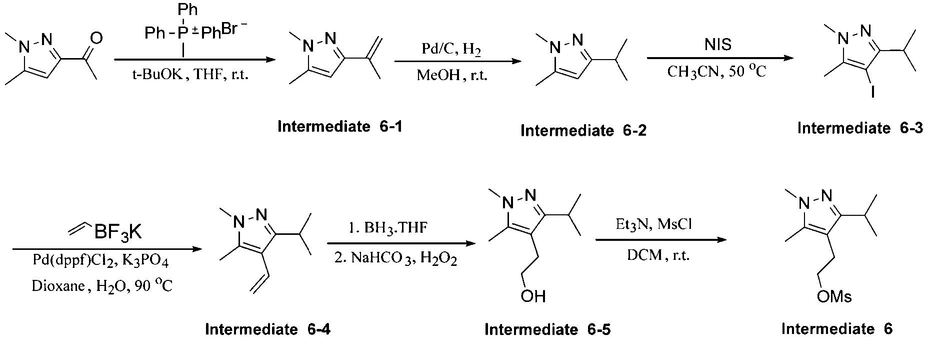

### Step 1: Synthesis of Intermediate 6-1

Methyltriphenylphosphonium bromide (6.21 g, 17.39 mmol) was dissolved in THF (50 mL). Potassium tert-butoxide (1.95 g, 17.39 mmol) was added at room temperature, and the mixture was stirred for 10 minutes. Subsequently, a solution of 1-(1,5-dimethyl-1H-pyrazol-3-yl)ethan-1-one (2 g, 14.49 mmol) in THF (10 mL) was added, and the reaction mixture was stirred at room temperature. The reaction progress was monitored by LC-MS, and the starting material was completely consumed. The mixture was poured into water (100 mL) and extracted with EA (50 mL*3). The combined organic phases were dried, concentrated, and purified by column chromatography (PE/EA = 20/1 to 5/1) to afford intermediate 6-1 as a yellow solid (1.54 g, yield 78%). ESI-MS m/z: 137.0 [M+H]⁺.

### Step 2: Synthesis of Intermediate 6-2

**Intermediate 6-1** (1.54 g, 11.32 mmol) was dissolved in methanol (50 mL). Pd/C (150 mg, 10%) was added, and the reaction system was purged with hydrogen. The mixture was stirred at room temperature, and the reaction progress was monitored by LC-MS until the starting material was completely consumed. The mixture was directly filtered with suction and concentrated to afford **intermediate 6-2** as a white solid (1.48 g, yield 95%). ESI-MS m/z: 139.0 [M+H]⁺.

Subsequently, using a synthetic method similar to that described in Preparation Example **1, Intermediate 6** can be obtained.

| Compound | Structure | Name | ESI-MS: [M+H]⁺ |
|---|---|---|---|
| **Intermediate 6** | 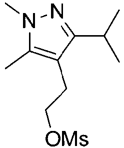 | 2-[1,5-dimethyl-3-(prop-2-yl)pyrazol -4-yl]ethyl methanesulfonate | 261.1 |

### Preparation Example 4: Synthesis of 2-(3-(fluoromethyl)-1,5-dimethylpyrazol -4-yl]ethyl methanesulfonate (Intermediate 7)

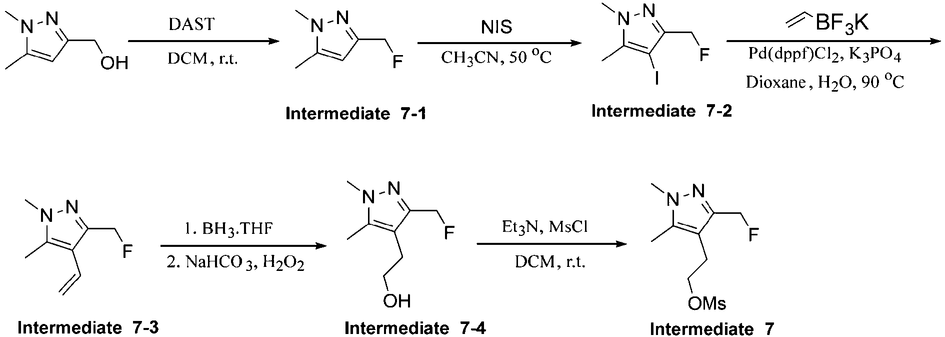

### Step 1: Synthesis of Intermediate 7-1

1,5-Dimethyl-1H-pyrazole-3-methanol (2 g, 15.87 mmol) was dissolved in DCM (60 mL). DAST (3.84 g, 23.81 mmol) was added at room temperature, and the reaction mixture was stirred at room temperature. The reaction progress was monitored by LC-MS, and the starting material was completely consumed. The mixture was poured into water (100 mL) and extracted with DCM (50 mL*3). The combined organic phases were dried, concentrated, and purified by column chromatography (PE/EA = 20:1 to 5:1) to afford **intermediate 7-1** as a yellow solid (1.65 g, yield 82%). ESI-MS m/z: 129.0 [M+H]⁺.

Subsequently, using a synthetic method similar to that described in Preparation Example 1, **Intermediate 7** can be obtained.

| Compound | Structure | Name | ESI-MS: [M+H]⁺ |
|---|---|---|---|
| **Intermediate 7** | 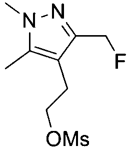 | 2-[3-(fluoromethyl)-1,5-dimethylpyra zol-4-yl]ethyl methanesulfonate | 251.0 |

### Preparation Example 5: Synthesis of 2-(3-(cyanomethyl)-1,5-dimethylpyrazol-4-yl]ethyl methanesulfonate (Intermediate 8)

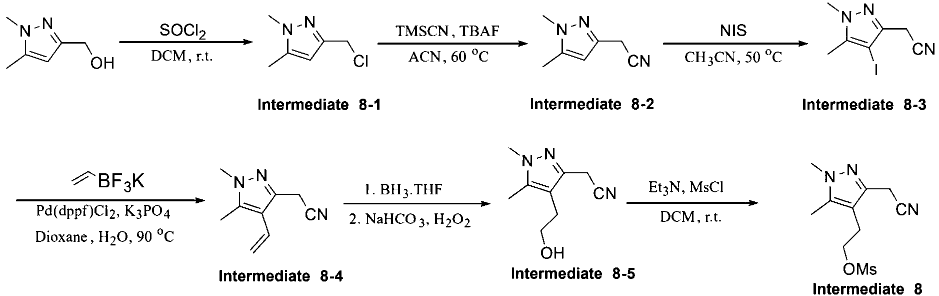

### Step 1: Synthesis of Intermediate 8 - 1

1,5-Dimethyl-1*H*-pyrazole-3-methanol **(2** g, 15.87 mmol) was dissolved in DCM (60 mL). Thionyl chloride (3.78 g, 31.74 mmol) was added at room temperature, and the reaction mixture was stirred at room temperature. The reaction progress was monitored by LC-MS until the starting material was completely consumed. The reaction mixture was directly concentrated to afford **intermediate 8-1** as a yellow solid (2.41 g, yield exceeding 100%). ESI-MS m/z: 145.0 [M+H]⁺.

### Step 2: Synthesis of Intermediate 8-2

**Intermediate 8-1** (2.41 g, 15.87 mmol) was dissolved in ACN (60 mL). TMSCN (3.15 g, 31.74 mmol) and TBAF (8.30 g, 31.74 mmol) were added at room temperature, and the reaction mixture was stirred at 60 °C. The reaction progress was monitored by LC-MS until the starting material was completely consumed. The reaction mixture was then poured into water (100 mL), extracted with EA (50 mL*3). The organic phases were combined, dried, concentrated, and purified by column chromatography (PE/EA = 10/1 to 5/1) to afford **intermediate 8-2** as a yellow solid (1.36 g, yield 63%). ESI-MS m/z: 136.0 [M+H]⁺.

Subsequently, using a synthetic method similar to that described in Preparation Example 1, **Intermediate 8** can be obtained.

| **Compound** | **Structure** | **Name** | **ESI-MS: [M+H]⁺** |
|---|---|---|---|
| **Intermediate 8** | 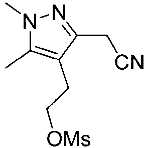 | 2-[3-(cyanomethyl)-1,5-dimethylpyra zol-4-yl]ethyl methanesulfonate | 258.0 |

### Example 1: Synthesis of 4-{2-[(5-chloro-2-{3-[(dimethylamino)methyl]-pyrazolo [1,5-a]pyridin-5-yl}phenyl)oxy]ethyl}-1,5,N,N-tetramethylpyrazole-3-carboxamide (Compound 1)

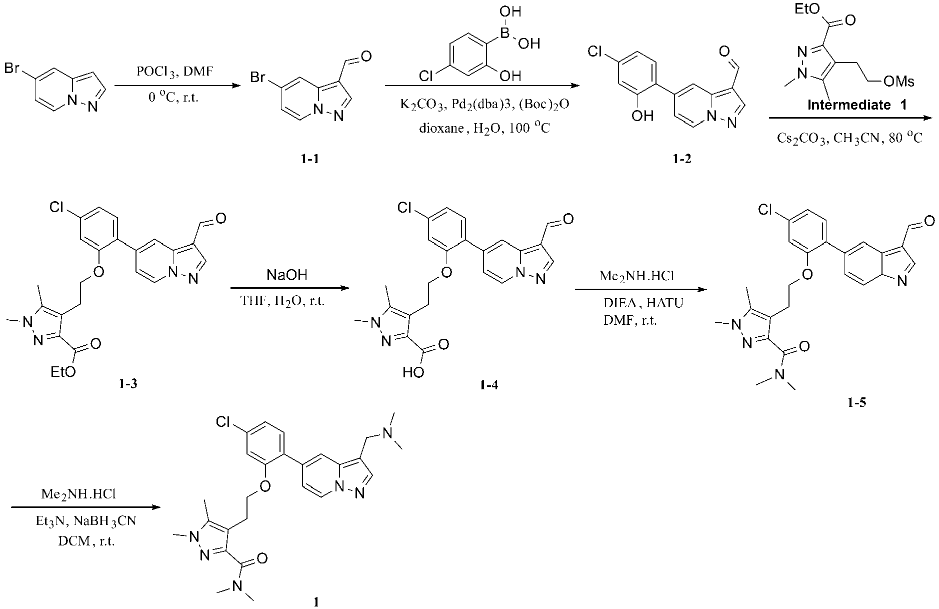

### Step 1: Synthesis of 1-1

5-Bromopyrazolo[1,5-a]pyridine (1 g, 5.08 mmol) was dissolved in DMF (20 mL). POCl₃ (2.33 g, 15.2 mmol) was slowly added at 0 °C. After the addition was complete, the reaction mixture was stirred at room temperature. The reaction progress was monitored by LC-MS until the starting material was completely consumed. The mixture was poured into water (30 mL), and the pH was adjusted to 10 with 1 M sodium hydroxide solution. The mixture was extracted with EA (30 mL* 3), and the combined organic phases were dried and concentrated to afford **1-1** as a white solid (965 mg, yield 77%). ESI-MS m/z: 224.9 [M+H]⁺.

### Step 2: Synthesis of 1-2

**1-1** (200 mg, 0.89 mmol) and 4-chloro-2-hydroxyphenylboronic acid (230 mg, 1.33 mmol) were dissolved in 1,4-dioxane/water (10/2 mL). Pd₂(dba)₃ (81.4 mg, 0.089 mmol), (Boc)₂O (582 mg, 2.67 mmol) and potassium carbonate (368 mg, 2.67 mmol) were added, and the reaction mixture was stirred at 100 °C. The reaction progress was monitored by LC-MS until the starting material was completely consumed. Water (30 mL) was added to the mixture, which was then extracted with EA (30 mL* 3). The combined organic phases were dried, concentrated, and purified by column chromatography (PE/EA = 10/1 to 3/1) to afford **1-2** as a yellow solid (211 mg, yield 87%). ESI-MS m/z: 272.9 [M+H]⁺.

### Step 3: Synthesis of 1-3

**1-2** (100 mg, 0.37 mmol) was dissolved in acetonitrile (10 mL). **Intermediate 1** (178 mg, 0.61 mmol) and Cs₂CO₃ (597 mg, 1.83 mmol) were added, and the reaction mixture was stirred at 80 °C. The reaction progress was monitored by LC-MS until the starting material was completely consumed. Water (30 mL) was added, and the mixture was extracted with EA (30 mL*3). The combined organic phases were dried, concentrated, and purified by column chromatography (PE/EA = 20/1 to 2/1) to afford **1-3** as a yellow solid (110 mg, yield 77%). ESI-MS m/z: 467.1 [M+H]⁺.

### Step 4: Synthesis of 1-4

1-3 (100 mg, 214 µmol) was dissolved in THF/water (5/0.5 mL). NaOH (12.9 mg, 321 µmol) was added, and the reaction mixture was stirred at room temperature. The reaction progress was monitored by LC-MS until the starting material was completely consumed. The mixture was poured into water (30 mL), and the pH was adjusted to 5-6 with 1 M hydrochloric acid. The mixture was extracted with EA (30 mL* 3), and the combined organic phases were dried and concentrated to afford 1-4 as a yellow solid (40 mg, crude). ESI-MS m/z: 439.0 [M+H]⁺.

### Step 5: Synthesis of 1-5

**1-4** (40 mg, 91.1 µmol) was dissolved in DMF (3 mL). Dimethylamine hydrochloride (14.9 mg, 182 µmol), DIEA (58.9 mg, 456 µmol) and HATU (41.6 mg, 109 µmol) were added sequentially, and the reaction mixture was stirred at room temperature. The reaction progress was monitored by LC-MS until the starting material was completely consumed. The mixture was poured into water (20 mL), extracted with EA (10 mL*3). The combined organic phases were dried and concentrated to afford **1-5** as a yellow solid (47 mg, crude). ESI-MS m/z: 465.1 [M+H]⁺.

### Step 6: Synthesis of Compound 1

**1-5** (47.0 mg, 101 µmol) was dissolved in DCM (5 mL). TEA (30.6 mg, 303 µmol), NaBH₃CN (7.61 mg, 121 µmol) and dimethylamine hydrochloride (24.7 mg, 303 µmol) were added, and the reaction mixture was stirred at room temperature. The reaction progress was monitored by LC-MS until the starting material was completely consumed. The mixture was poured into water (20 mL), extracted with EA (20 mL*3). The combined organic phases were dried, concentrated, and purified by preparative chromatography to afford **1** as a yellow solid (14 mg, yield 28%).

¹H NMR (400 MHz, CD₃OD): δ 8.61 (d, *J =* 7.25 Hz, 1H), 8.19 (s, 1H), 7.92-7.95 (m, 1H), 7.44 (d, *J =* 8.13 Hz, 1H), 7.17-7.21 (m, 1H), 7.10 (br d, *J*=1.88 Hz, 2H), 4.55-4.61 (m, 2H), 4.20 (t, *J =* 6.69 Hz, 2H), 3.68-3.71 (m, 3H), 2.97-3.07 (m, 6H), 2.93-2.97 (m, 2H), 2.92 (s, 6H), 2.05 (s, 3H); ESI-MS m/z: 495.1 [M+H]⁺.

### Examples 2-27: Synthesis of Compounds 2-11 and 13-28

**Compounds 2-11 and 13-28** can be synthesized by a method similar to **Example 1.**

| Compound | Structure | Name | ESI-MS: [M+H]⁺ |
|---|---|---|---|
| **2** | 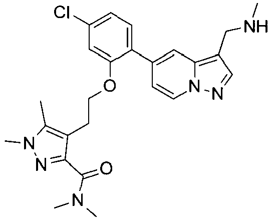 | 4-{2-[(5-chloro-2-{3-[(methylamino) methyl]pyrazolo[1,5-a]pyridin-5-yl}p henyl)oxy]ethyl}-1,5,N,N-tetramethyl pyrazole-3-carboxamide | 481.2 |
| **3** | 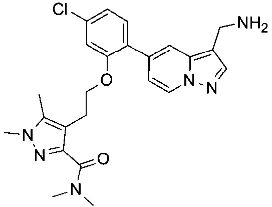 | 4-[2-({2-[3-(aminomethyl)pyrazolo[1, 5-a]pyridin-5-yl]-5-chlorophenyl}oxy )ethyl]-1,5,N,N-tetramethylpyrazole-3-carboxamide | 467.1 |
| **4** | 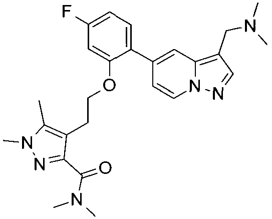 | 4-{2-[(2-{3-[(dimethylamino)methyl] pyrazolo[1,5-a]pyridin-5-yl}-5-fluoro phenyl)oxy]ethyl } -1 ,5,N,N-tetrameth ylpyrazole-3-carboxamide | 479.2 |
| **5** | 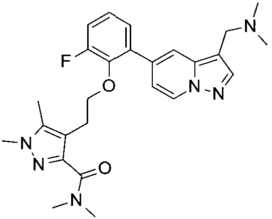 | 4-{2-[(6-{3-[(dimethylamino)methyl] pyrazolo[1,5-a]pyridin-5-yl}-2-fluoro phenyl)oxy]ethyl } -1 ,5,N,N-tetrameth ylpyrazole-3-carboxamide | 479.2 |
| **6** | 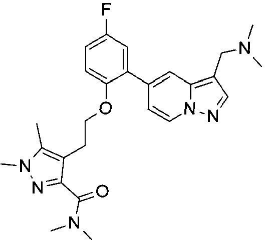 | 4-{2-[(2-{3-[(dimethylamino)methyl] pyrazolo[1,5-a]pyridin-5-yl}-4-fluoro phenyl)oxy]ethyl}-1,5,N,N-tetrameth ylpyrazole-3-carboxamide | 479.2 |
| **7** | 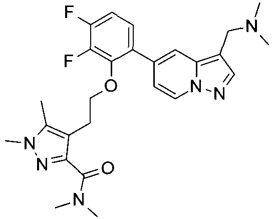 | 4-{2-[(6-{3-[(dimethylamino)methyl] pyrazolo[1,5-a]pyridin-5-yl}-2,3-diflu orophenyl)oxy]ethyl}-1,5,N,N-tetram ethylpyrazole-3-carboxamide | 497.2 |
| **8** | 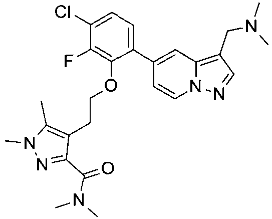 | 4-{2-[(3-chloro-6-{3-[(dimethylamin o)methyl]pyrazolo[1,5-a]pyridin-5-yl }-2-fluorophenyl)oxy]ethyl}-1,5,N,N-tetramethylpyrazole-3-carboxamide | 513.2 |
| **9** | 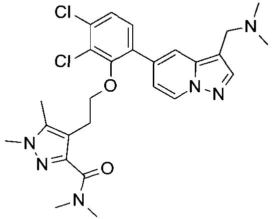 | 4-{2-[(2,3-dichloro-6-{3-[(dimethyla mino)methyl]pyrazolo[1,5-a]pyridin-5-yl}phenyl)oxy]ethyl}-1,5,N,N-tetra methylpyrazole-3-carboxamide | 529.1 |
| **10** | 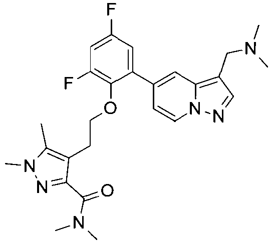 | 4-{2-[(6-{3-[(dimethylamino)methyl] pyrazolo[1,5-a]pyridin-5-yl}-2,4-diflu orophenyl)oxy]ethyl}-1,5,N,N-tetram ethylpyrazole-3-carboxamide | 497.2 |
| **11** | 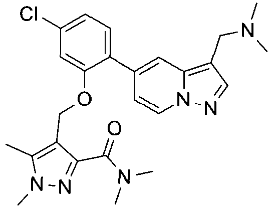 | 4-{[(5-chloro-2-{3-[(dimethylamino) methyl]pyrazolo[1,5-a]pyridin-5-yl}p henyl)oxy]methyl}-1,5,N,N-tetrameth ylpyrazole-3-carboxamide | 481.2 |
| **13** | 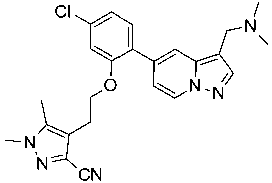 | 4-{2-[(5-chloro-2-{3-[(dimethylamin o)methyl]pyrazolo[1,5-a]pyridin-5-yl }phenyl)oxy]ethyl}-1,5-dimethylpyra zole-3-carbonitrile | 449.1 |
| **14** | 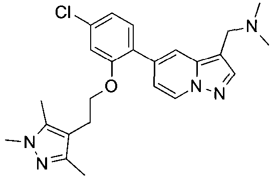 | 5-(4-chloro-2-{[2-(1,3,5-trimethylpyr azol-4-yl)ethyl]oxy}phenyl)-3-[(dime thylamino)methyl]pyrazolo[1,5-a]pyri dine | 438.1 |
| **15** | 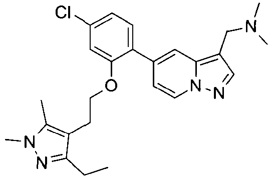 | 5-(4-chloro-2-{[2-(3-ethyl-1,5-dimeth ylpyrazol-4-yl)ethyl]oxy}phenyl)-3-[( dimethylamino)methyl]pyrazolo[1,5-a]pyridine | 452.2 |
| **16** | 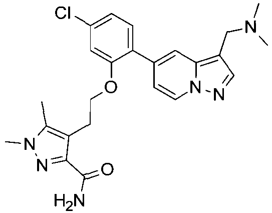 | 4-{2-[(5-chloro-2-{3-[(dimethylamin o)methyl]pyrazolo[1,5-a]pyridin-5-yl }phenyl)oxy]ethyl}-1,5-dimethylpyra zole-3-carboxamide | 467.1 |
| **17** | 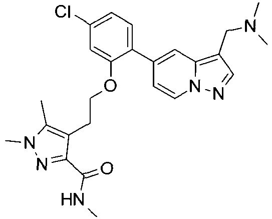 | 4-{2-[(5-chloro-2-{3-[(dimethylamin o)methyl]pyrazolo[1,5-a]pyridin-5-yl }phenyl)oxy]ethyl}-1,5,N-trimethylp yrazole-3-carboxamide | 481.2 |
| **18** | 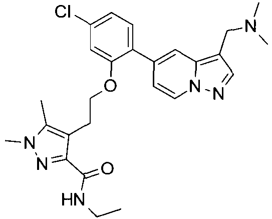 | 4-{2-[(5-chloro-2-{3-[(dimethylamin o)methyl]pyrazolo[1,5-a]pyridin-5-yl }phenyl)oxy]ethyl}-N-ethyl-1,5-dime thylpyrazole-3-carboxamide | 495.2 |
| **19** | 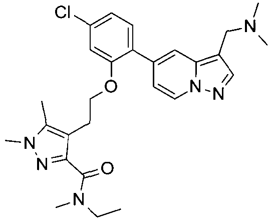 | 4-{2-[(5-chloro-2-{3-[(dimethylamin o)methyl]pyrazolo[1,5-a]pyridin-5-yl }phenyl)oxy]ethyl}-N-ethyl-1,5,N-tri methylpyrazole-3-carboxamide | 509.2 |
| **20** | 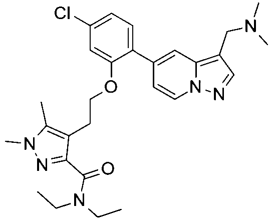 | 4-{2-[(5-chloro-2-{3-[(dimethylamin o)methyl]pyrazolo[1,5-a]pyridin-5-yl }phenyl)oxy]ethyl}-N,N-diethyl-1,5-dimethylpyrazole-3-carboxamide | 523.2 |
| **21** | 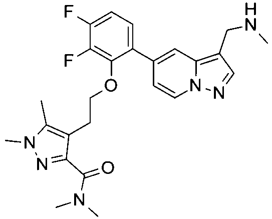 | 4-{2-[(2,3-difluoro-6-{3-[(methylami no)methyl]pyrazolo[1,5-a]pyridin-5-y 1}phenyl)oxy]ethyl}-1,5,N,N-tetramet hylpyrazole-3-carboxamide | 483.1 |
| **22** | 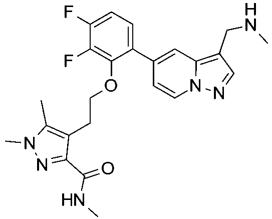 | 4-{2-[(2,3-difluoro-6-{3-[(methylami no)methyl]pyrazolo[1,5-a]pyridin-5-y l}phenyl)oxy]ethyl}-1,5,N-trimethylp yrazole-3-carboxamide | 469.1 |
| **23** | 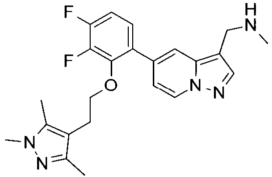 | 5-(3,4-difluoro-2-{[2-(1,3,5-trimethyl pyrazol-4-yl)ethyl]oxy}phenyl)-3-[(m ethylamino)methyl]pyrazolo[1,5-a]py ridine | 426.1 |
| **24** | 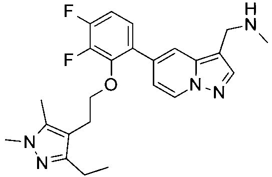 | 5-(2-{[2-(3-ethyl-1,5-dimethylpyrazol -4-yl)ethyl]oxy}-3,4-difluorophenyl)-3-[(methylamino)methyl]pyrazolo[1,5 -a]pyridine | 440.1 |
| **25** | 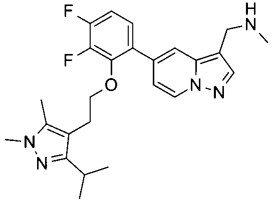 | 5-[2-({2-[1,5-dimethyl-3-(prop-2-yl)p yrazol-4-yl]ethyl}oxy)-3,4-difluoroph enyl]-3-[(methylamino)methyl]pyrazo lo[1,5-a]pyridine | 454.1 |
| **26** | 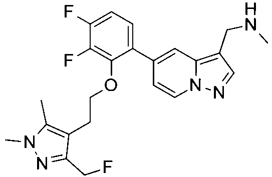 | 5-[3,4-difluoro-2-({2-[3-(fluoromethy l)-1,5-dimethylpyrazol-4-yl]ethyl}ox y)phenyl]-3-[(methylamino)methyl]p yrazolo[1,5-a]pyridine | 444.1 |
| **27** | 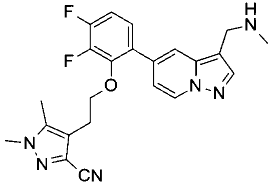 | 4-{2-[(2,3-difluoro-6-{3-[(methylami no)methyl]pyrazolo[1,5-a]pyridin-5-y l}phenyl)oxy]ethyl}-1,5-dimethylpyr azole-3-carbonitrile | 437.1 |
| **28** | 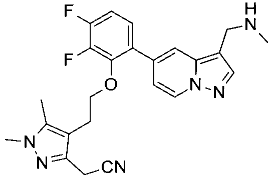 | (4-{2-[(2,3-difluoro-6-{3-[(methylam ino)methyl]pyrazolo[1,5-a]pyridin-5-yl}phenyl)oxy]ethyl}-1,5-dimethylpy razol-3-yl)acetonitrile | 451.1 |

### Example 28: Synthesis of (4-{2-[(5-chloro-2-{3-[(dimethylamino)methyl]-pyrazolo [1,5-a]pyridin-5-yl}phenyl)oxy]ethyl}-1,5-dimethylpyrazol-3-yl)methanol (Compound 12)

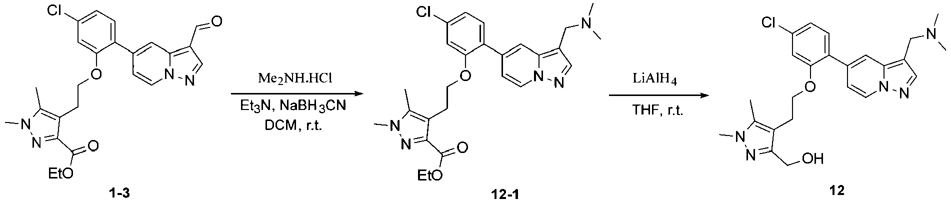

### Step 1: Synthesis of 12-1

**1-3** (200 mg, 0.43 mmol) was dissolved in DCM (10 mL). TEA (130 mg, 1.29 mmol), NaBH₃CN (81 mg, 1.29 mmol) and dimethylamine hydrochloride (105 mg, 1.29 mmol) were added, and the reaction mixture was stirred at room temperature. The reaction progress was monitored by LC-MS until the starting material was completely consumed. The mixture was poured into water (20 mL), extracted with EA (20 mL*3). The combined organic phases were dried and concentrated to afford 12-1 as a yellow solid (220 mg, crude). ESI-MS m/z: 496.1 [M+H]⁺.

### Step 2: Synthesis of Compound 12

**12-1** (220 mg, 0.43 mmol) was dissolved in THF (10 mL). LiAlH₄ (33 mg, 0.86 mmol) was added, and the reaction mixture was stirred at room temperature. The reaction progress was monitored by LC-MS until the starting material was completely consumed. The reaction was quenched with saturated aqueous ammonium chloride solution, then extracted with EA (20 mL*3). The combined organic phases were dried, concentrated, and purified by preparative chromatography to afford 12 as a yellow solid (84 mg, yield 43%).

¹H NMR (400 MHz, CD₃OD): δ 8.64 (d, *J =* 7.22 Hz, 1H), 8.16 (s, 1H), 7.92-7.97 (m, 1H), 7.41 (d, *J =* 8.11 Hz, 1H), 7.22 (m, 1H), 7.12 (br d, *J =* 1.82 Hz, 2 H), 4.56 (m, 2H), 4.35 (s, 2 H), 4.22 (t, *J* = 6.61 Hz, 2H), 3.68-3.73 (m, 3H), 3.05 (s, 6H), 2.90 (m, 2H), 2.01 (s, 3H); ESI-MS m/z: 454.1 [M+H]⁺.

### Example 29: Synthesis of 2-(4-{2-[(2,3-difluoro-6-{3-[(methylamino)methyl]-pyrazolo[1,5-a]pyridin-5-yl}phenyl)oxy]ethyl}-1,5-dimethylpyrazol-3-yl)propan-2-ol (Compound 29)

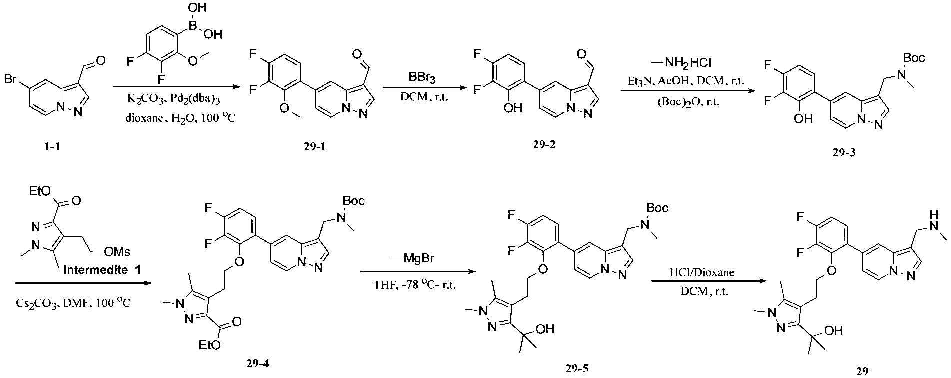

### Step 1: Synthesis of 29-1

**1-1** (500 mg, 2.22 mmol) and 3,4-difluoro-2-methoxyphenylboronic acid (418 mg, 2.22 mmol) were dissolved in 1,4-dioxane/water (30 mL/3 mL). Pd₂(dba)₃ (204 mg, 0.22 mmol) and K₂CO₃ (613 mg, 4.44 mmol) were added, and the reaction mixture was stirred at 100 °C. The reaction progress was monitored by LC-MS until the starting material was completely consumed. Water (30 mL) was added, and the mixture was extracted with EA (30 mL*3). The combined organic phases were dried, concentrated, and purified by column chromatography (PE/EA = 10/1 to 2/1) to afford **29-1** as a yellow solid (420 mg, yield 66%). ESI-MS m/z: 288.9 [M+H]⁺.

### Step 2: Synthesis of 29-2

**29-1** (420 mg, 1.46 mmol) was dissolved in DCM (30 mL). BBr₃ (366 mg, 4.37 mmol) was added at room temperature, and the reaction mixture was stirred at room temperature. The reaction progress was monitored by LC-MS until the starting material was completely consumed. Water (30 mL) was slowly added to quench the reaction, and the mixture was neutralized with saturated sodium bicarbonate solution. The mixture was extracted with EA (30 mL*3), and the combined organic phases were dried, concentrated, and purified by column chromatography (PE/EA = 5/1 to 1/1) to afford **29-2** as a yellow solid (290 mg, yield 73%). ESI-MS m/z: 274.9 [M+H]⁺.

### Step 3: Synthesis of 29-3

**29-2** (290 mg, 1.06 mmol) was dissolved in DCM (20 mL). TEA (321 mg, 3.18 mmol), AcOH (64 mg, 1.06 mmol), methylamine hydrochloride (143 mg, 2.12 mmol) and NaBH₃CN (333 mg, 5.30 mmol) were added, and the reaction mixture was stirred at room temperature. The reaction progress was monitored by LC-MS until the starting material was completely consumed. Subsequently, (Boc)₂O (462 mg, 2.12 mmol) was added, and the mixture was stirred at room temperature. The reaction progress was monitored by LC-MS until the starting material was completely consumed. The mixture was poured into water (30 mL), extracted with DCM (20 mL*3). The combined organic phases were dried, concentrated, and purified by column chromatography (PE/EA = 5/1 to 1/1) to afford **29-3** as a yellow solid (310 mg, yield 75%). ESI-MS m/z: 390.1 [M+H]⁺.

### Step 4: Synthesis of 29-4

**29-3** (310 mg, 0.80 mmol) was dissolved in DMF (30 mL). **Intermediate 1** (279 mg, 0.96 mmol) and Cs₂CO₃ (521 mg, 1.60 mmol) were added, and the reaction mixture was stirred at 100 °C. The reaction progress was monitored by LC-MS until the starting material was completely consumed. Water (30 mL) was added, and the mixture was extracted with EA (30 mL* 3). The combined organic phases were dried, concentrated, and purified by column chromatography (PE/EA = 20/1 to 3/1) to afford **29-4** as a yellow solid (260 mg, yield 56%). ESI-MS m/z: 584.2 [M+H]⁺.

### Step 5: Synthesis of 29-5

**29-4** (260 mg, 0.45 mmol) was dissolved in THF (20 mL). Methylmagnesium bromide (1 M, 1.35 mL, 1.35 mmol) was added at -78 °C, and then the reaction mixture was slowly warmed to room temperature and stirred. The reaction progress was monitored by LC-MS until the starting material was completely consumed. The mixture was poured into water (30 mL), extracted with EA (20 mL × 3). The combined organic phases were dried, concentrated, and purified by column chromatography (PE/EA = 5/1 to 1/1) to afford 29-5 as a yellow solid (130 mg, yield 51%). ESI-MS m/z: 570.2 [M+H]⁺.

### Step 6: Synthesis of 29

**29-5** (130 mg, 0.23 mmol) was dissolved in DCM (10 mL). HCl in 1,4-dioxane (4 M, 1 mL) was added, and the reaction mixture was stirred at room temperature. The reaction progress was monitored by LC-MS until the starting material was completely consumed. The

mixture was directly concentrated, and purified by preparative chromatography to afford 29 as a yellow solid (60 mg, yield 56%).

¹H NMR (400 MHz, DMSO-*d*₆): δ 8.66 (d, *J =* 7.8 Hz, 1H), 8.18 (s, 1H), 8.03 (s, 1H), 7.41-7.29 (m, 2H), 7.09 (dd, *J =* 7.2, 1.8 Hz, 1H), 4.69 (s, 1H), 4.34 (s, 2H), 4.00 (t, *J =* 7.8 Hz, 2H), 3.51 (s, 3H), 2.88-2.82 (m, 2H), 2.55 (s, 3H), 1.86 (s, 3H), 1.29 (s, 6H); ESI-MS m/z: 470.1 [M+H]⁺.

### Examples 30: Synthesis of Compound 33

**Compound 33** can be synthesized by a method similar to **Example 29.**

| Compound | Structure | Name | ESI-MS: [M+H]⁺ |
|---|---|---|---|
| **33** | 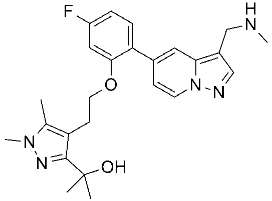 | 2-(4-{2-[(5-fluoro-2-{3-[(methyl amino)methyl]pyrazolo[1,5-a]pyridin-5-yl}phenyl)oxy]ethyl}-1,5-dimethylpyrazol-3-yl)propan-2-ol | 452.1 |

### Example 31: Synthesis of 1-(4-{2-[(2,3-difluoro-6-{3-[(methylamino)methyl]-pyrazolo[1,5-a]pyridin-5-yl}phenyl)oxy]ethyl}-1,5-dimethylpyrazol-3-yl)-2,2-dimethylpr opan-1-ol (Compound 30)

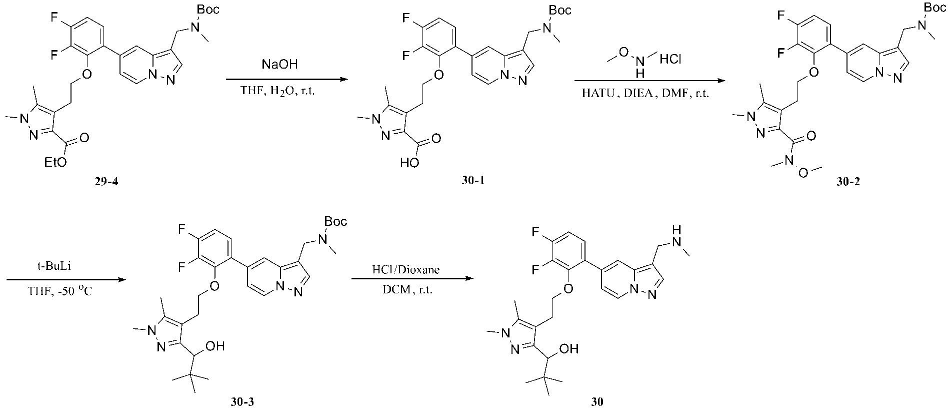

### Step 1: Synthesis of 30-1

**29-4** (200 mg, 0.34 mmol) was dissolved in THF/water (10/1 mL). NaOH (27 mg, 0.69 mmol) was added, and the reaction mixture was stirred at room temperature. The reaction progress was monitored by LC-MS until the starting material was completely consumed. The mixture was poured into water (30 mL), and the pH was adjusted to 5-6 with 1 M hydrochloric acid. The mixture was extracted with EA (30 mL*3), and the combined organic phases were dried and concentrated to afford **30-1** as a yellow solid (170 mg, yield 90%). ESI-MS m/z: 556.1 [M+H]⁺.

### Step 2: Synthesis of 30-2

**30-1** (170 mg, 0.31 mmol) was dissolved in DMF (10 mL). N,O-Dimethylhydroxylamine hydrochloride (45 mg, 0.47 mmol), DIEA (80 mg, 0.62 mmol) and HATU (179 mg, 0.47 mmol) were added sequentially, and the reaction mixture was stirred at room temperature. The reaction progress was monitored by LC-MS until the starting material was completely consumed. The mixture was poured into water (30 mL), extracted with EA (20 mL*3). The combined organic phases were dried, concentrated, and purified by column chromatography (PE/EA = 5/1 to 1/1) to afford **30-2** as a yellow solid (160 mg, yield 86%). ESI-MS m/z: 599.2 [M+H]⁺.

### Step 3: Synthesis of 30-3

**30-2** (160 mg, 0.27 mmol) was dissolved in THF (20 mL). ^{t-}BuLi (1.3 M, 0.25 mL, 0.32 mmol) was added at -50 °C, and then the reaction mixture was slowly warmed to room temperature and stirred. The reaction progress was monitored by LC-MS until the starting material was completely consumed. The mixture was poured into water (30 mL), extracted with EA (20 mL*3). The combined organic phases were dried, concentrated, and purified by column chromatography (PE/EA = 5/1 to 0/1) to afford 30-3 as a yellow solid (70 mg, yield 44%). ESI-MS m/z: 598.2 [M+H]⁺.

### Step 4: Synthesis of Compound 30

**30-3** (70 mg, 0.12 mmol) was dissolved in DCM (10 mL). HCl in 1,4-dioxane (4 M, 1 mL) was added, and the reaction mixture was stirred at room temperature. The reaction progress was monitored by LC-MS until the starting material was completely consumed. The mixture was directly concentrated, and purified by preparative chromatography to afford 30 as a yellow solid (32 mg, yield 53%).

¹H NMR (400 MHz, DMSO-*d*₆): δ 8.65 (d, *J =* 7.3 Hz, 1H), 8.30 (s, 1H), 8.07 (s, 1H), 7.90 (s, 1H), 7.37-7.30 (m, 2H), 7.03 (dd, *J=* 7.2, 1.8 Hz, 1H), 4.10 (d, *J =* 7.9 Hz, 3H), 3.86 (dd, *J =* 15.7, 9.1 Hz, 2H), 2.72-2.57 (m, 2H), 1.87 (s, 3H), 1.23 (d, *J =* 6.7 Hz, 2H), 0.89 (s, 3H), 0.78 (s, 9H); ESI-MS m/z: 498.1 [M+H]⁺.

### Examples 32: Synthesis of Compound 34

**Compound 34** can be synthesized by a method similar to **Example 30.**

| Compound | Structure | Name | ESI-MS: [M+H]⁺ |
|---|---|---|---|
| **34** | 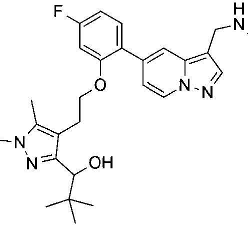 | 1-(4-{2-[(5-fluoro-2-{3-[(methylamino)methyl]pyrazolo[1,5-*a*]pyridin-5-yl}phenyl)oxy]ethyl}-1,5-dimethylpyrazol-3-yl)-2,2-dimethylpropan-1-ol | 480.2 |

### Example 33: Synthesis of 1-(4-{2-[(2,3-difluoro-6-{3-[(methylamino)methyl]-pyrazolo[1,5-a]pyridin-5-yl}phenyl)oxy]ethyl}-1,5-dimethylpyrazol-3-yl)ethan-1-ol (Compound 31)

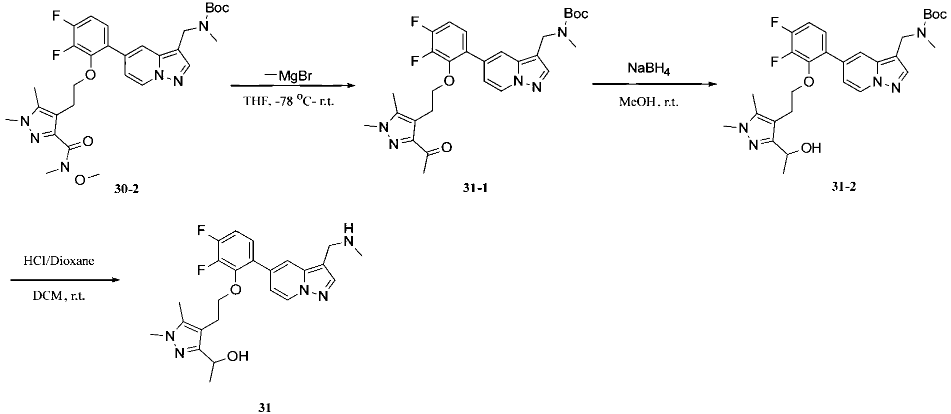

### Step 1: Synthesis of 31-1

**30-2** (200 mg, 0.33 mmol) was dissolved in THF (20 mL). Methylmagnesium bromide (1 M, 0.40 mL, 0.40 mmol) was added at -78 °C, and then the reaction mixture was slowly warmed to room temperature and stirred. The reaction progress was monitored by LC-MS until the starting material was completely consumed. The mixture was poured into water (30 mL), extracted with EA (20 mL*3). The combined organic phases were dried, concentrated, and purified by column chromatography (PE/EA = 5/1 to 1/1) to afford 31-1 as a yellow solid (110 mg, yield 59%). ESI-MS m/z: 554.2 [M+H]⁺.

### Step 2: Synthesis of 31-2

**31-1** (110 mg, 0.20 mmol) was dissolved in methanol (10 mL). NaBH₄ (11 mg, 0.30 mmol) was added at room temperature, and the reaction mixture was stirred at room temperature. The reaction progress was monitored by LC-MS until the starting material was completely consumed. The mixture was poured into water (30 mL), extracted with EA (20 mL*3). The combined organic phases were dried, concentrated, and purified by column chromatography (PE/EA = 5/1 to 0/1) to afford **31-2** as a yellow solid (85 mg, yield 77%). ESI-MS m/z: 556.2 [M+H]⁺.

### Step 3: Synthesis of 31

**31-2** (85 mg, 0.15 mmol) was dissolved in DCM (10 mL). HCl in 1,4-dioxane (4 M, 1 mL) was added, and the reaction mixture was stirred at room temperature. The reaction progress was monitored by LC-MS until the starting material was completely consumed. The mixture was directly concentrated, and purified by preparative chromatography to afford 31 as a yellow solid (22 mg, yield 33%).

¹H NMR (400 MHz, DMSO-*d*₆): δ 8.61 (d, *J =* 7.7 Hz, 1H), 8.19 (s, 1H), 8.05 (s, 1H), 7.41-7.26 (m, 2H), 7.11 (m, 1H), 4.66 (s, 1H), 4.31 (s, 2H), 4.01 (t, *J =* 7.8 Hz, 2H), 3.81 (m, 1H), 3.52 (s, 3H), 2.88-2.80 (m, 2H), 2.57 (s, 3H), 1.88 (s, 3H), 1.25 (s, 3H); ESI-MS m/z: 446.1 [M+H]⁺.

### Examples 34-36: Synthesis of Compound 32,35,36

**Compounds 32, 35, 36** can be synthesized by a method similar to **Example 31.**

| Compound | Structure | Name | ESI-MS: [M+H]⁺ |
|---|---|---|---|
| **32** | 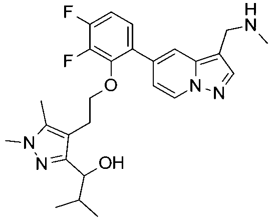 | 1-(4- {2-[(2,3-difluoro-6- {3-[(methylamino)methyl]pyrazolo[1,5-*a*]pyridin-5-yl}phenyl)oxy]ethyl}-1,5-dimethylpyrazol-3-yl)-2-methylpropan-1-ol | 484.2 |
| **35** | 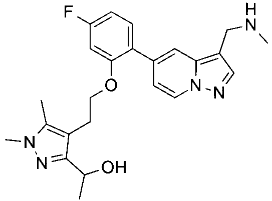 | 1-(4-{2-[(5-fluoro-2-{3-[(methyl amino)methyl]pyrazolo[1,5-*a*]pyridin-5-yl}phenyl)oxy]ethyl}-1,5-dimethylpyrazol-3-yl)ethan-1-ol | 438.2 |
| **36** | 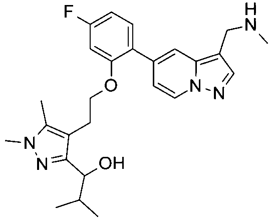 | 1-(4-{2-[(5-fluoro-2-{3-[(methyl amino)methyl]pyrazolo[1,5-*a*]pyridin-5-yl}phenyl)oxy]ethyl}-1,5-dimethylpyrazol-3-yl)-2-methylpropan-1-ol | 466.2 |

### Example 37: Screening of Compound Inhibitory Activity on NMT1 Enzyme Catalysis

This study evaluated the ability of the compounds of the invention to inhibit NMT1 catalytic activity *in vitro.* The experiment used a FI (Fluorescence Intensity) analysis method, characterizing NMT1 inhibitors under conditions of 1.25 µM myristoyl-CoA (Lithium salt, Sigma, M4414). In this experiment, a synthesized peptide with the sequence H-Gly-Ser-Asn-Lys-Ser-Lys-Pro-Lys-NH₂, HSPP60SRC (Nanjing Peptide Industry, NJP23953), was used as the reaction substrate. The final concentration of the NMT1 enzyme was 6 nM, the final concentration of CPM (Reactive thiol fluorescence probe, Invitrogen, D346) was 1 µM, the final concentration of HSPP60 SRC was 20 µM, and the final concentration of DMSO was 0.5%.

Compounds were dissolved in DMSO to obtain 10 mM stock solutions. Dose-response solutions were prepared with a top endpoint compound concentration of 3 mM, followed by three-fold serial dilutions in DMSO, totaling ten data points. Using an ECHO acoustic liquid handler (BECKMAN, ECHO 655 system), 0.05 µL of the diluted compound solutions was transferred to a 384-well assay plate (Corning, CLS4514). Then, 2.5 µL of the NMT1 enzyme working solution was added to the 384-well assay plate, followed by incubation at 25 °C for 10 minutes. Subsequently, 5 µL of the CPM and myristoyl-CoA working solution was added, the plate was centrifuged at 1000 rpm for 1 minute, and then 2.5 µL of the HSPP60 SRC working solution was added to initiate the reaction. The reaction was incubated at 25 °C for 45 minutes. The FI signal (em: 320 nm; ex: 405 nm) was detected using an HTS high-throughput screening multifunctional microplate reader (BMG, PHERA star FSX).

The inhibition rate of the compounds on NMT1 enzyme catalytic activity was calculated using the following formula: Inhibition Percentage (%) = 100 * (Mean of DMSO group - Mean of Compound group) / (Mean of DMSO group - Mean of Blank control group). The IC₅₀ curve of the compounds was fitted using the software XLfit 5.5.0 according to the nonlinear regression equation. Table 1 provides the inhibitory activity of the compounds of the invention on NMT1 enzyme catalysis.

### Example 38: Screening of Compound Anti-proliferative Activity against MV-4-11 Cells

This study utilized a cell proliferation assay to analyze the cytotoxicity of MV-4-11 human monocytic leukemia cells after three days of exposure to NMT1 inhibitors. The MV-4-11 cell line was purchased from Nanjing Cobuilder Biotechnology Co., Ltd., and cultured in IMDM medium (Viva cell) in an incubator (Thermo) at 37 °C with 5% CO₂. In this experiment, NMT1 inhibitors were dissolved in DMSO to obtain 600 µM stock solutions. Dose-response solutions were prepared with a top endpoint compound concentration of 3 µM, followed by three-fold serial dilutions in DMSO, totaling eight data points. The final concentration of DMSO was 0.5%.

MV-4-11 cells were seeded into a white 96-well plate, 80 µL of cell suspension per well, containing 6000 MV-4-11 cells. The cell plate was placed in the CO₂ incubator for overnight culture. Then, 20 µL of NMT1 inhibitor solutions at different concentrations were added to the wells. The 96-well plate was placed back into the incubator for three days. Another cell plate was prepared, and the signal was read on the day of drug addition to serve as the maximum value (Max value in the equation below) for data analysis. To this cell plate, 25 µL of cell viability chemiluminescence detection reagent was added per well, followed by incubation at room temperature for 10 minutes to stabilize the luminescence signal. A multi-label analyzer was used for reading.

The raw data were converted into inhibition rates using the equation (Sample - Min) / (Max - Min) * 100%. The IC₅₀ values were then determined by fitting a four-parameter curve (using the "log(inhibitor) vs. response - Variable slope" model in GraphPad Prism). Min: wells treated with 0.5% DMSO; Max: Day 0 wells. Table 1 provides the inhibitory activity of the compounds of the invention on MV-4-11 cell proliferation.

**Table 1: Compound Inhibitory Activity against NMT1 Enzyme (IC₅₀) and Anti-proliferative Activity against MV-4-11 Cells (IC₅₀)**

| Compound | NMT1 (IC₅₀, nM) | MV-4-11 (IC₅₀, nM) | Compound | NMT1 (IC₅₀, nM) | MV-4-11 (IC₅₀, nM) |
|---|---|---|---|---|---|
| **1** | 1.77 | 17.08 | **19** | 3.21 | 29.78 |
| **2** | 2.13 | 23.36 | **20** | 2.76 | 23.47 |
| **3** | 3.45 | 41.2 | **21** | 1.61 | 5.91 |
| **4** | 1.65 | 11.42 | **22** | 1.89 | 10.56 |
| **5** | 1.74 | 11.85 | **23** | 2.57 | 19.44 |
| **6** | 2.89 | 30.21 | **24** | 2.81 | 24.92 |
| **7** | 2.10 | 19.38 | **25** | 3.26 | 25.77 |
| **8** | 2.89 | 28.42 | **26** | 2.48 | 12.29 |
| **9** | 3.68 | 32.47 | **27** | 1.82 | 5.56 |
| **10** | 2.59 | 26.43 | **28** | 1.95 | 10.74 |
| **11** | 6.31 | 58.73 | **29** | 1.70 | 3.15 |
| **12** | 3.67 | 31.74 | **30** | 1.62 | <1.37 |
| **13** | 2.79 | 23.21 | **31** | 1.82 | 4.79 |
| **14** | 1.98 | 18.35 | **32** | 1.71 | 3.93 |
| **15** | 2.34 | 20.68 | **33** | 2.13 | 5.49 |
| **16** | 5.32 | 41.82 | **34** | 1.93 | 3.87 |
| **17** | 2.38 | 25.78 | **35** | 2.05 | 5.04 |
| **18** | 2.97 | 30.16 | **36** | 1.96 | 4.89 |

From the data in the table above, it can be seen that the compounds of the present invention possess strong inhibitory activity against NMT1 enzyme catalysis, and also exhibit potent anti-proliferative activity against MV-4-11 cells. In particular, compounds such as **21, 27, 29, 30, 31, 32, 34** and **36** demonstrate very strong inhibitory activity against NMT1 enzyme catalysis and anti-proliferative activity against MV-4-11 cells, with IC₅₀ values for NMT1 enzyme catalytic inhibition less than 2 nM and IC₅₀ values for anti-proliferative activity against MV-4-11 cells less than 10 nM.

All references mentioned in the present disclosure are cited as references in this application, just as each reference is cited separately. In addition, it should be understood that after reading the above lecture content of the present disclosure, those skilled in the art can make various changes or modifications to the present disclosure, and these equivalent forms also fall within the scope of the claims attached to the present disclosure.

## Claims

1. A compound having the structure shown in Formula (1), or an isomer, crystal form, pharmaceutically acceptable salt, hydrate, or solvate thereof: 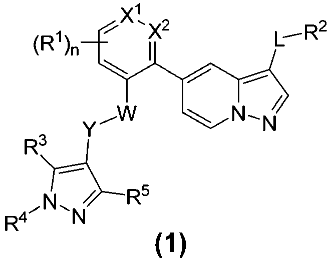 wherein:
n is 0, 1, 2 or 3;
X¹ and X² are each independently selected from the group consisting of: CH, CR¹ or N;
W is selected from the group consisting of: a bond, O, S or NH;
Y is selected from the group consisting of: a bond, -(CH₂)ₘ-, -(CH₂)ₘO- or -(CH₂)ₘO-(CH₂)ₘ, wherein m is 1, 2 or 3;
L is -(CH₂)ₘ-;
each R¹ is independently selected from the group consisting of: D, hydroxyl, halogen, CN, NO₂, NH₂, C₁₋₄ alkyl, C₂₋₄ alkenyl, C₂₋₄ alkynyl, C₁₋₄ haloalkyl, C₁₋₄ alkoxy, C₁₋₄ haloalkoxy, C₃₋₆ saturated or partially unsaturated carbocyclic group, 3-8-membered saturated or partially unsaturated heterocyclyl, C₆₋₁₀ aryl or 5-12 membered heteroaryl;
R² is -NR⁶R⁷, wherein R⁶ and R⁷ are each independently selected from the group consisting of: H, D, C₁₋₆ alkyl or C₁₋₆ haloalkyl;
or, R⁶ and R⁷ are taken together with the attached nitrogen atom to form a 3-8-membered saturated or partially unsaturated heterocyclyl, or a 5-12 membered heteroaryl;
R³ is selected from the group consisting of: H, D, C₁₋₄ alkyl, C₁₋₄ haloalkyl, C₁₋₄ alkoxy, C₁₋₄ haloalkoxy, C₃₋₆ saturated or partially unsaturated carbocyclic group, 3-8-membered saturated or partially unsaturated heterocyclyl, C₆₋₁₀ aryl or 5-12 membered heteroaryl;
R⁴ is selected from the group consisting of: C₁₋₄ alkyl, C₁₋₄ haloalkyl, C₃₋₆ saturated or partially unsaturated carbocyclic group, 3-8-membered saturated or partially unsaturated heterocyclyl, C₆₋₁₀ aryl or 5-12 membered heteroaryl;
R⁵ is selected from the group consisting of: hydroxyl, CN, C₁₋₆ alkyl, C₁₋₆ haloalkyl, hydroxyl substituted C₁₋₆ alkyl, cyano substituted C₁₋₆ alkyl or -C(O)NR⁸R⁹; wherein R⁸ and R⁹ are each independently selected from the group consisting of: H, D, C₁₋₆ alkyl or C₁₋₆ haloalkyl;
or, R⁸ and R⁹ are taken together with the attached nitrogen atom to form a 3-8-membered saturated or partially unsaturated heterocyclyl, or a 5-12 membered heteroaryl;
wherein, the alkyl, alkenyl, alkynyl, haloalkyl, alkoxy, haloalkoxy, carbocyclic group, heterocyclyl, aryl and heteroaryl are each optionally substituted by one or more substituents selected from the group consisting of: D, hydroxyl, halogen, CN, NO₂, NH₂, C₁₋₄ alkyl, C₂₋₄ alkenyl, C₂₋₄ alkynyl, C₁₋₄ haloalkyl, C₁₋₄ alkoxy or C₁₋₄ haloalkoxy.

2. The compound according to claim 1, or an isomer, crystal form, pharmaceutically acceptable salt, hydrate, or solvate thereof, wherein each R¹ is independently selected from the group consisting of: F, Cl, Br, CN, NO₂, C₁₋₃ alkyl, C₁₋₃ haloalkyl, C₁₋₃ alkoxy or haloalkoxy.

3. The compound according to claim 1, or an isomer, crystal form, pharmaceutically acceptable salt, hydrate, or solvate thereof, wherein R² is -NR⁶R⁷, wherein R⁶ and R⁷ are each independently selected from the group consisting of: H, D, C₁₋₃ alkyl or C₁₋₃ haloalkyl.

4. The compound according to claim 1, or an isomer, crystal form, pharmaceutically acceptable salt, hydrate, or solvate thereof, wherein R³ is selected from the group consisting of: H, D, C₁₋₃ alkyl or C₁₋₃ haloalkyl.

5. The compound according to claim 1, or an isomer, crystal form, pharmaceutically acceptable salt, hydrate, or solvate thereof, wherein R⁴ is selected from the group consisting of: C₁₋₃ alkyl or C₁₋₃ haloalkyl.

6. The compound according to claim 1, or an isomer, crystal form, pharmaceutically acceptable salt, hydrate, or solvate thereof, wherein R⁵ is selected from the group consisting of: CN, C₁₋₆ alkyl, C₁₋₆ haloalkyl, hydroxyl substituted C₁₋₆ alkyl, cyano substituted C₁₋₆ alkyl or -C(O)NR⁸R⁹;
wherein R⁸ and R⁹ are each independently selected from the group consisting of: H, D, C₁₋₃ alkyl or C₁₋₃ haloalkyl.

7. The compound according to claim 1, an isomer thereof, a crystal form, or a pharmaceutically acceptable salt thereof, wherein the compound has a structure selected from the group consisting of:

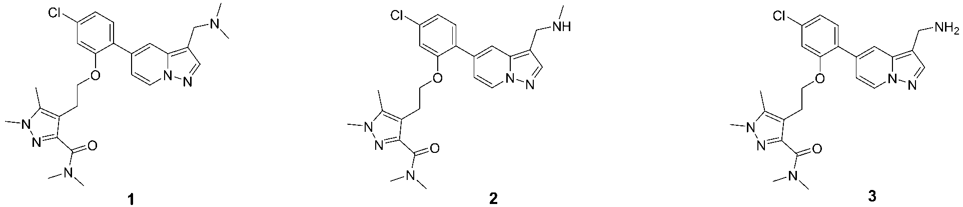

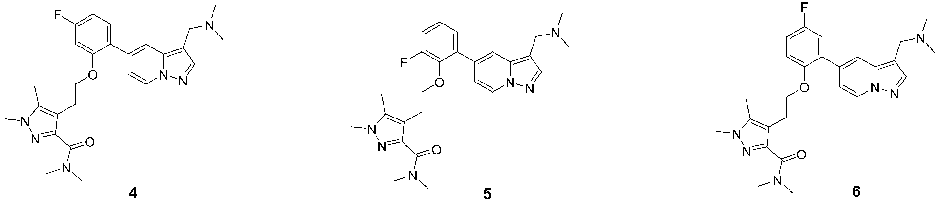

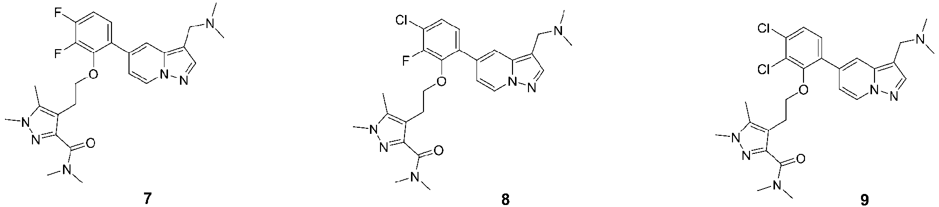

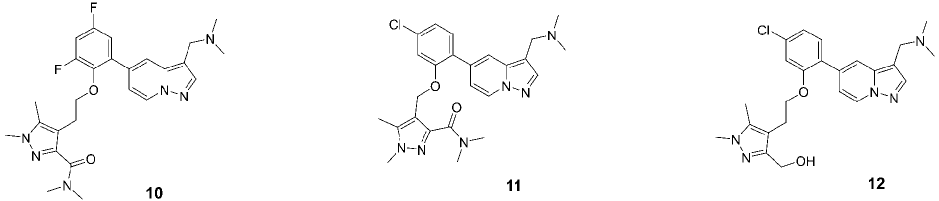

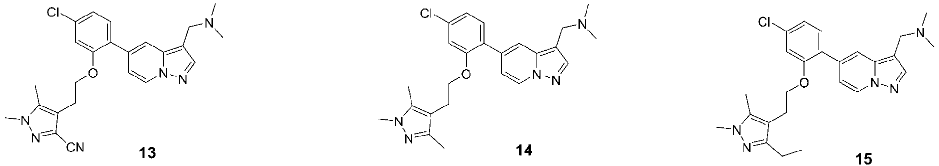

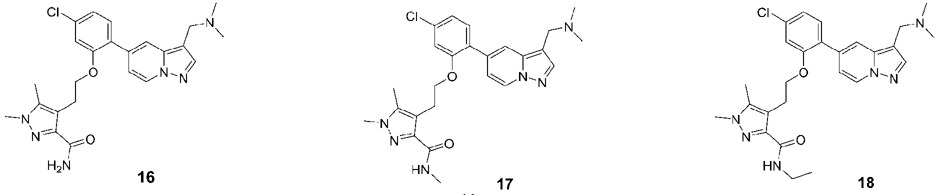

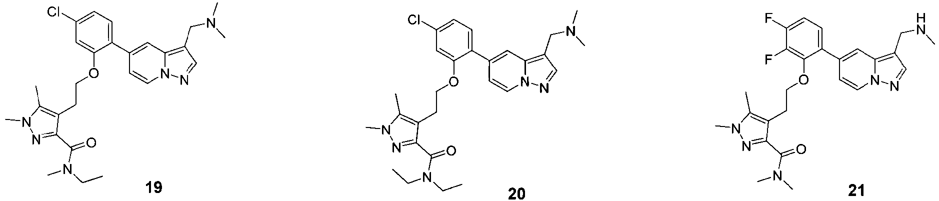

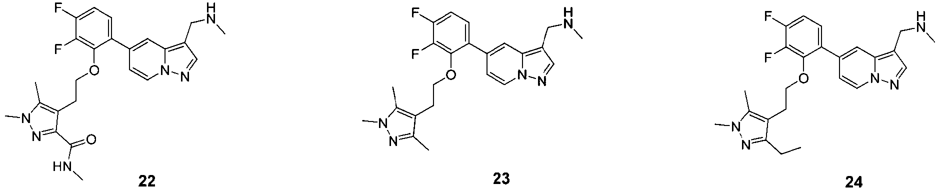

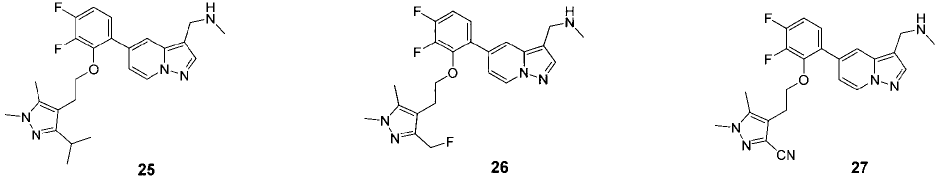

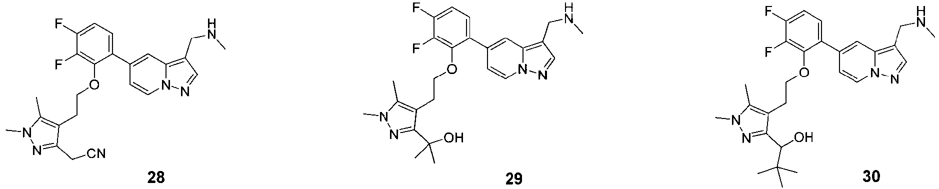

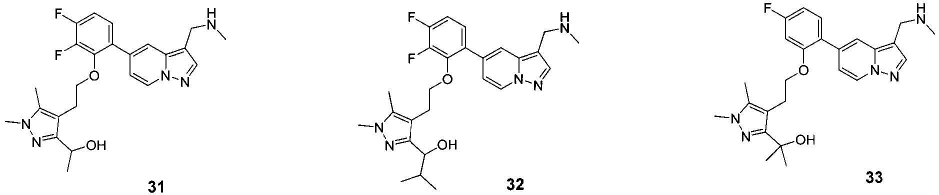

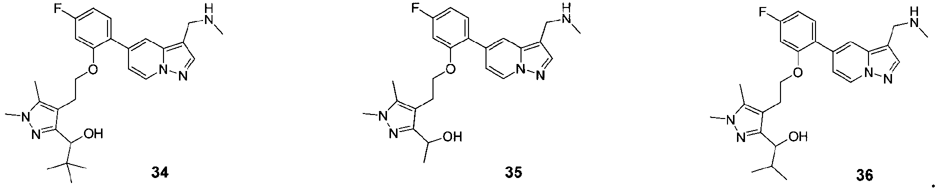

8. A pharmaceutical composition for treating, regulating, and/or preventing a disease associated with NMT, **characterized in that** the pharmaceutical composition comprises:
(1) the compound as an active ingredient according to any one of claims 1-7, or an isomer, crystal form, pharmaceutically acceptable salt, hydrate, or solvate thereof; and
optionally (2) a pharmaceutically acceptable excipient or carrier.

9. Use of the compound according to any one of claims 1-7, or an isomer, crystal form, pharmaceutically acceptable salt, hydrate or solvate thereof, or the pharmaceutical composition according to claim 8, for preparing a pharmaceutical composition for treating, regulating and/or preventing a disease mediated by NMT.

10. A method for treating, regulating, and/or preventing a disease mediated by NMT, **characterized by** comprising the steps of: administering to an individual in need with a compound according to any one of claims 1-7, or an isomer, crystal form, pharmaceutically acceptable salt, hydrate, or solvate, or a pharmaceutical composition according to claim 8.

11. The use according to claim 9 or the method according to claim 10, wherein the related disease mediated by NMT includes infectious disease or hyperproliferative disease.

12. The use or method according to claim 11, wherein the infectious disease includes protozoan infection and viral infection; preferably, the protozoan infection includes malaria and leishmaniasis; preferably, the viral infection includes human rhinovirus infection and HIV infection.

13. The use or method according to claim 11, wherein the hyperproliferative disease is selected from the following group: lymphoma, leukemia, brain tumor, gastric tumor, liver cancer, lung cancer, intestinal cancer, pancreatic cancer, breast cancer, cervical cancer, ovarian cancer, endometrial cancer and prostate cancer.
